# EUROPEAN PATENT APPLICATION

(11) **EP 4 732 805 A2**
(43) Date of publication of application: **29.04.2026**
(21) Application number: 26154248.4
(22) Date of filing: 20.05.2022
(51) Int. Cl.: A61B 34/20

(54) **OPTIMIZATION OF TRACKER-BASED SURGICAL NAVIGATION**

(30) Priority: 20.05.2021 US 202163190791 P
(62) Divisional of application: 25194501.0
(71) Applicant: Mako Surgical Corp., Weston, FL 33331 (US)
(72) Inventor: UMBDENSTOCK, Emeric, 79106 Freiburg (DE); HOEKSTRA, Paul, Kalamazoo, 49009 (US); SCHWOERER, Trudbert, 77978 Schuttertal (DE); RIEGELSBERGER, Fabian, 79224 Umkirch (DE); ROHS, Helmut, 79110 Freiburg (DE); WOODS, Philip Robert, Kalamazoo, 49009 (US)
(74) Representative: Schott, Jakob Valentin

(57) **Abstract**

Systems and methods for optimizing tracking an object in a surgical workspace. A tracker is disposed relative to the object that includes a predefined geometry of markers for tracking a pose of the tracker in the surgical workspace. A localizer camera cooperates with the tracker to generate image data indicating a blob for each of the markers generated from a light signal received from the marker. A characteristic of each blob is acquired, and the acquired characteristics are compared to an optimal characteristic. Based on the comparison, the operation of the trackers, the localizer, or both are adjusted to optimize the blobs generated from the markers.

## Description

### CROSS REFERENCE TO RELATED APPLICATIONS

This application claims priority to U.S. Provisional Application No. 63/190,791 filed on May 20, 2021, the disclosure of which is hereby incorporated by reference herein in its entirety.

### BACKGROUND

Conventional surgical navigation systems track objects in a surgical workspace by imaging fiducials mounted to the objects and computing the position of such fiducials in the surgical workspace from the imaging. Suboptimal lighting can impact a surgical navigation system's ability to precisely determine the position of each fiducial, which in turn may impact its tracking precision.

### SUMMARY

This Summary introduces a selection of concepts in a simplified form that are further described in the Detailed Description below. This Summary is not intended to limit the scope of the claimed subject matter, and does not necessarily identify each and every key or essential feature of the claimed subject matter.

In a first aspect, a navigation system for optimizing tracking of an object in a surgical workspace is provided. The navigation system comprises a tracker disposed relative to the object and including a predefined geometry of active markers for tracking a pose of the tracker in the surgical workspace, a localizer camera configured to cooperate with the tracker to generate image data indicating a blob for each of the active markers generated from a light signal emitted from the active marker, and a controller communicatively coupled to the tracker and the localizer camera. The controller is configured to assign each of the blobs to the active marker corresponding to the blob; acquire a characteristic of each blob; compare the acquired characteristics to an optimal characteristic; and based on the comparison, communicate at least one control signal to the tracker that causes the tracker to adjust the light signal emitted from at least one of the active markers.

In a second aspect, a navigation system for optimizing tracking of objects in a surgical workspace is provided. The navigation system includes a first tracker disposed relative to a first object in the surgical workspace and including a predefined geometry of active markers for tracking a pose of the first tracker in the surgical workspace, a second tracker disposed relative to a second object in the surgical workspace and including a predefined geometry of active markers for tracking a pose of the second tracker in the surgical workspace, a localizer camera configured to cooperate with the first and second trackers to generate image data indicating a first blob for each of the active markers of the first tracker generated from a light signal emitted from the active marker and a second blob for each of the active markers of the second tracker generated from a light signal emitted from the active marker, and a controller communicatively coupled to the first and second trackers and the localizer camera. The controller is configured to acquire a characteristic of each of the first and second blobs; compare the acquired characteristics to a first optimal characteristic specific to the first tracker and a second optimal characteristic specific to the second tracker that differs from the first optimal characteristic; and based on the comparison, assign the first blobs to the first tracker and the second blobs to the second tracker.

In a third aspect, a navigation system for optimizing tracking of an object in a surgical workspace is provided. The navigation system includes a tracker disposed relative to the object and including a predefined geometry of active markers for tracking a pose of the tracker in the surgical workspace, a localizer camera configured to cooperate with the tracker to generate image data indicating a blob for each of the active markers generated from a light signal emitted from the active marker, and a controller communicatively coupled to the tracker and the localizer camera. The controller is configured to determine positions of the active markers of the tracker in the surgical workspace based on the image data; and based on the determined positions of the active markers, communicate at least one control signal to the tracker that causes the tracker to adjust the light signal emitted from at least one of the active markers.

In a fourth aspect, a navigation system for optimizing tracking of an object in a surgical workspace is provided. The navigation system includes a tracker disposed relative to the object and including a predefined geometry of passive markers for tracking a pose of the tracker in the surgical workspace, a localizer camera including a light source configured to emit a light signal for illuminating the passive markers, the localizer camera being configured to generate image data indicating a blob for each of the passive markers generated from a reflection by the passive marker of the light signal emitted from the light source, and a controller communicatively coupled to the localizer camera. The controller is configured to acquire a characteristic of each blob; compare the acquired characteristics to an optimal characteristic; and based on the comparison, adjust at least one optical parameter of the localizer camera.

In a fifth aspect, a navigation system for tracking objects in a surgical workspace is provided. The navigation system includes a first tracker disposed relative to a first object in the surgical workspace and including a predefined geometry of passive markers for tracking a pose of the first tracker in the surgical workspace, a second tracker disposed relative to a second object in the surgical workspace and including a predefined geometry of passive markers for tracking a pose of the second tracker in the surgical workspace, a localizer camera including a light source configured to emit a light signal for illuminating the passive markers of the first and second trackers, the localizer camera being configured to generate image data indicating a blob for each of the passive markers of the first and second trackers generated from a reflection by the passive marker of the light signal emitted from the light source, and a controller communicatively coupled to the localizer camera. The controller is configured to emit a first light signal from the light source that is specific to the first tracker; receive image data generated by the localizer camera corresponding to the emitted first light signal; and track a pose of the first tracker in the surgical workspace based on the received image data corresponding to the first light signal. The controller is further configured to emit a second light signal from the light source specific to the second tracker and having at least one characteristic that differs from at least one corresponding characteristic of the first light signal; receive image data generated by the localizer camera corresponding to the emitted second light signal; and track a pose of the second tracker in the surgical workspace based on the received image data corresponding to the second light signal.

In a sixth aspect, a navigation system for optimizing tracking of an object in a surgical workspace is provided. The navigation system includes a tracker disposed relative to the object and including a predefined geometry of passive markers for tracking a pose of the tracker in the surgical workspace; a localizer camera including a light source configured to emit a light signal for illuminating the passive markers, the localizer camera being configured to generate image data indicating a blob for each of the passive markers generated from a reflection by the passive marker of the light signal emitted from the light source; and a controller communicatively coupled to the localizer camera. The controller is configured to emit light signals from the light source having varying characteristics; receive image data generated by the localizer camera for each of the emitted light signals that indicates a blob for each of the passive markers generated from a reflection by the passive marker of the emitted light signal; for each instance of received image data, acquire a characteristic of each blob indicated by the image data and compare the acquired characteristics to an optimal characteristic to determine which of the instances of received image data is closest to optimal; responsive to determining the instance of received image data closest to optimal, assign the characteristics of the light signal corresponding to the instance of received image data to the tracker; and track a pose of the tracker in the surgical workspace based on the light signal characteristics assigned to the tracker.

In a seventh aspect, a navigation system for optimizing tracking of an object in a surgical workspace is provided. The navigation system includes a tracker disposed relative to the object and including a predefined geometry of passive markers for tracking a pose of the tracker in the surgical workspace; a localizer camera including a light source configured to emit a light signal for illuminating the passive markers, the localizer camera being configured to generate image data indicating a blob for each of the passive markers generated from a reflection by the passive marker of the light signal emitted from the light source; and a controller communicatively coupled to the localizer camera. The controller is configured to determine positions of the passive markers of the tracker in the surgical workspace based on the image data; and based on the determined positions of the passive markers, adjust at least one optical parameter of the localizer camera.

In an eighth aspect, a navigation system for optimizing tracking of an object in a surgical workspace is provided. The navigation system includes a tracker disposed relative to the object and including a predefined geometry of manually repositionable passive markers for tracking a pose of the tracker in the surgical workspace; a localizer camera including a light source configured to emit a light signal for illuminating the passive markers, the localizer camera being configured to generate image data indicating a blob for each of the passive markers generated from a reflection by the passive marker of the light signal emitted from the light source; and a controller communicatively coupled to the localizer camera. The controller is configured to acquire a characteristic of each blob; compare the acquired characteristics to an optimal characteristic; and determine and display guidance for repositioning the passive markers of the tracker based on the comparison.

In a ninth aspect, a method for optimizing tracking of an object in a surgical workspace by a navigation system is provided. The navigation system includes a tracker disposed relative to the object and including a predefined geometry of active markers for tracking a pose of the tracker in the surgical workspace, a localizer camera configured to cooperate with the tracker to generate image data indicating a blob for each of the active markers generated from a light signal emitted from the active marker, and a controller communicatively coupled to the tracker and the localizer camera. The method comprises disposing the tracker relative to the object in the surgical workspace; generating, by the localizer camera, the image data indicating a blob for each of the active markers generated from the light signal emitted from the active marker; assigning, by the controller, each of the blobs to the active marker corresponding to the blob; acquiring, by the controller, a characteristic of each blob; comparing, by the controller, the acquired characteristics to an optimal characteristic; and based on the comparison, communicating, by the controller, at least one control signal to the tracker that causes the tracker to adjust the light signal emitted from at least one of the active markers.

In a tenth aspect, a method for optimizing tracking of an object in a surgical workspace by a navigation system is provided. The navigation system includes a first tracker disposed relative to a first object in the surgical workspace and including a predefined geometry of active markers for tracking a pose of the first tracker in the surgical workspace, a second tracker disposed relative to a second object in the surgical workspace and including a predefined geometry of active markers for tracking a pose of the second tracker in the surgical workspace, a localizer camera configured to cooperate with the first and second trackers to generate image data indicating a blob for each of the active markers of the first and second trackers generated from a light signal emitted from the active marker, and a controller communicatively coupled to the first and second trackers and the localizer camera. The method includes disposing the first and second trackers relative to the first and second objects respectively in the surgical workspace; generating, by the localizer camera, the image data indicating a blob for each of the active markers of the first and second trackers generated from a light signal emitted from the active marker; acquiring, by the controller, a characteristic of each of the first and second blobs; comparing, by the controller, the acquired characteristics to a first optimal characteristic specific to the first tracker and a second optimal characteristic specific to the second tracker that differs from the first optimal characteristic; and based on the comparison, assigning, by the controller, the first blobs to the first tracker and the second blobs to the second tracker.

In an eleventh aspect, a method for optimizing tracking of an object in a surgical workspace by a navigation system is provided. The navigation system includes a tracker disposed relative to the object and including a predefined geometry of active markers for tracking a pose of the tracker in the surgical workspace, a localizer camera configured to cooperate with the tracker to generate image data indicating a blob for each of the active markers generated from a light signal emitted from the active marker, and a controller communicatively coupled to the tracker and the localizer camera. The method includes disposing the tracker relative to the object in the surgical workspace; generating, by the localizer camera, the image data indicating a blob for each of the active markers generated from a light signal emitted from the active marker; determining, by the controller, positions of the active markers of the tracker in the surgical workspace based on the image data; and based on the determined positions of the active markers, communicating, by the controller, at least one control signal to the tracker that causes the tracker to adjust the light signal emitted from at least one of the active markers.

In a twelfth aspect, a method is provided for optimizing tracking of an object in a surgical workspace by a navigation system. The navigation system includes a tracker disposed relative to the object and including a predefined geometry of passive markers for tracking a pose of the tracker in the surgical workspace, a localizer camera including a light source configured to emit a light signal for illuminating the passive markers, the localizer camera being configured to generate image data indicating a blob for each of the passive markers generated from a reflection by the passive marker of the light signal emitted from the light source, and a controller communicatively coupled to the localizer camera. The method comprises disposing the tracker relative to the object in the surgical workspace; generating, by the localizer camera, the image data indicating a blob for each of the passive markers generated from a reflection by the passive marker of the light signal emitted from the light source; acquiring, by the controller, a characteristic of each blob; comparing, by the controller, the acquired characteristics to an optimal characteristic; and based on the comparison, adjusting, by the controller, at least one optical parameter of the localizer camera.

In a thirteenth aspect, a method is provided for tracking objects in a surgical workspace by a navigation system. The navigation system includes a first tracker disposed relative to a first object in the surgical workspace and including a predefined geometry of passive markers for tracking a pose of the first tracker in the surgical workspace, a second tracker disposed relative to a second object in the surgical workspace and including a predefined geometry of passive markers for tracking a pose of the second tracker in the surgical workspace, a localizer camera including a light source configured to emit a light signal for illuminating the passive markers of the first and second trackers, the localizer camera being configured to generate image data indicating a blob for each of the passive markers of the first and second trackers generated from a reflection by the passive marker of the light signal emitted from the light source, and a controller communicatively coupled to the localizer camera. The method comprises disposing the first and second trackers relative to the first and second objects respectively in the surgical workspace; emitting, from the light source, a first light signal specific to the first tracker; receiving, by the controller, image data generated by the localizer camera corresponding to the emitted first light signal; and tracking, by the controller, a pose of the first tracker in the surgical workspace based on the received image data corresponding to the first light signal. The method further comprises emitting, from the light source, a second light signal specific to the second tracker and having at least one characteristic that differs from at least one corresponding characteristic of the first light signal; receiving, by the controller, image data generated by the localizer camera corresponding to the emitted second light signal; and tracking, by the controller, a pose of the second tracker in the surgical workspace based on the received image data corresponding to the second light signal.

In a fourteenth aspect, a method is provided for optimizing tracking of an object in a surgical workspace by a navigation system. The navigation system includes a tracker disposed relative to the object and including a predefined geometry of passive markers for tracking a pose of the tracker in the surgical workspace; a localizer camera including a light source configured to emit a light signal for illuminating the passive markers, the localizer camera being configured to generate image data indicating a blob for each of the passive markers generated from a reflection by the passive marker of the light signal emitted from the light source; and a controller communicatively coupled to the localizer camera. The method includes disposing the tracker relative to the object in the surgical workspace; emitting, from the light source, light signals having varying characteristics; receiving, by the controller, image data generated by the localizer camera for each of the emitted light signals that indicates a blob for each of the passive markers generated from a reflection by the passive marker of the emitted light signal; for each instance of received image data, acquiring, by the controller, a characteristic of each blob indicated by the image data and comparing, by the controller, the acquired characteristics to an optimal characteristic to determine which of the instances of received image data is closest to optimal; responsive to determining the instance of received image data closest to optimal, assigning, by the controller, the characteristics of the light signal corresponding to the instance of received image data to the tracker; and tracking, by the controller, a pose of the tracker in the surgical workspace based on the light signal characteristics assigned to the tracker.

In a fifteenth aspect, a method is provided for optimizing tracking of an object in a surgical workspace by a surgical navigation system. The navigation system includes a tracker disposed relative to the object and including a predefined geometry of passive markers for tracking a pose of the tracker in the surgical workspace; a localizer camera including a light source configured to emit a light signal for illuminating the passive markers, the localizer camera being configured to generate image data indicating a blob for each of the passive markers generated from a reflection by the passive marker of the light signal emitted from the light source; and a controller communicatively coupled to the localizer camera. The method includes disposing the tracker relative to the object in the surgical workspace; generating, by the localizer camera, the image data indicating a blob for each of the passive markers generated from a reflection by the passive marker of the light signal emitted from the light source; determining, by the controller, positions of the passive markers of the tracker in the surgical workspace based on the image data; and based on the determined positions of the passive markers, adjusting, by the controller, at least one optical parameter of the localizer camera.

In a sixteenth aspect, a method is provided for optimizing tracking of an object in a surgical workspace by a navigation system. The navigation system includes a tracker disposed relative to the object and including a predefined geometry of repositionable passive markers for tracking a pose of the tracker in the surgical workspace; a localizer camera including a light source configured to emit a light signal for illuminating the passive markers, the localizer camera being configured to generate image data indicating a blob for each of the passive markers generated from a reflection by the passive marker of the light signal emitted from the light source; and a controller communicatively coupled to the localizer camera. The method includes disposing the tracker relative to the object in the surgical workspace; generating, by the localizer camera, the image data indicating a blob for each of the passive markers generated from a reflection by the passive marker of the light signal emitted from the light source; acquiring, by the controller, a characteristic of each blob; comparing, by the controller, the acquired characteristics to an optimal characteristic; and determining and displaying, by the controller, guidance for repositioning the passive markers of the tracker based on the comparison.

In a seventeenth aspect, a robotic surgical system is provided, comprising: a robotic device configured to support a surgical tool; and one or more controllers configured to implement the method of any one or more of the ninth through sixteenth aspects, wherein the one or more controllers are configured to control the robotic device to move the surgical tool relative to a cutting boundary to remove a target volume of patient tissue.

Any of the above aspects may be combined in-whole or in part.

Any of the aspects above may be utilized with any one or more of the following implementations, whether utilized individually or in combination:

Some implementations comprise the at least one control signal communicated to the tracker causing the tracker to adjust an intensity and/or duration of the light signal emitted from the at least one of the active markers. Some implementations comprise for each of the blobs comparing the acquired characteristic of the blob to the optimal characteristic to determine whether the blob is suboptimal; and responsive to determining that the blob is suboptimal based on the comparison, communicate a control signal to the tracker that causes the tracker to adjust the light signal emitted from the active marker corresponding to the blob.

Some implementations comprise the acquired characteristic of each blob indicating a first value, the optimal characteristic indicating a second value, and comparing the first value indicated for the blob to the second value; responsive to the comparison indicating that the first value for the blob is greater than the second value, communicating a control signal to the tracker that causes the tracker to reduce an intensity and/or duration of the light signal emitted from the active marker corresponding to the blob; and responsive to the comparison indicating that the first value for the blob is less than the second value, communicating a control signal to the tracker that causes the tracker to increase the intensity and/or duration of the light signal emitted from the active marker corresponding to the blob.

Some implementations comprise the acquired characteristics being blob intensity characteristics, and the optimal characteristic being an optimal blob intensity characteristic. Some implementations comprise the optimal blob intensity characteristic indicating an intensity value greater than or equal to 75% and less than or equal to 95% of a full scale intensity value of the localizer camera. Some implementations comprise the acquired characteristics being blob size characteristics, and the optimal characteristic being an optimal blob size characteristic. Some implementations comprise the acquired characteristics being blob shape characteristics, and the optimal characteristic being an optimal blob shape characteristic.

Some implementations comprise the acquired characteristics being defined as acquired first characteristics, the optimal characteristic being defined as a first optimal characteristic, and acquiring one or more second characteristics of one or more of the blobs; comparing the one or more acquired second characteristics to a second optimal characteristic; and based on the comparison of the one or more acquired second characteristics to the second optimal characteristic, communicating at least one control signal to the tracker that causes the tracker to adjust the light signal emitted from at least one of the one or more active markers corresponding to the one or more blobs. Some implementations comprise the one or more acquired second characteristics including an acquired second characteristic of each of the one or more blobs, and for each of the one or more blobs, comparing the acquired second characteristic of the blob to the second optimal characteristic to determine whether the blob is suboptimal; and responsive to determining that the blob is suboptimal based on the comparison, communicating a control signal to the tracker that causes the tracker to adjust the light signal emitted from the active marker corresponding to the blob.

Some implementations comprise the acquired characteristics being defined as acquired first characteristics, the optimal characteristic being defined as a first optimal characteristic, and for each blob, comparing the acquired first characteristic of the blob to the first optimal characteristic to determine whether the acquired first characteristic of the blob is suboptimal; responsive to determining that the acquired first characteristic of the blob is suboptimal based on the comparison, communicating a control signal to the tracker that causes the tracker to adjust the light signal emitted from the active marker corresponding to the blob; and responsive to determining that the acquired first characteristic of the blob is not suboptimal based on the comparison: acquiring a second characteristic of the blob; comparing the acquired second characteristic of the blob to a second optimal characteristic to determine whether the acquired second characteristic of the blob is suboptimal; and responsive to determining that the acquired second characteristic of the blob is suboptimal based on the comparison, communicating a control signal to the tracker that causes the tracker to adjust the light signal emitted from the active marker corresponding to the blob.

Some implementations comprise the acquired first characteristics being blob intensity characteristics, and the acquired second characteristics being blob size characteristics or blob shape characteristics. Some implementations comprise the acquired first characteristics being blob size characteristics, and the acquired second characteristics being blob intensity characteristics or blob shape characteristics. Some implementations comprise the acquired first characteristics being blob shape characteristics, and the acquired second characteristics being blob intensity characteristics or blob size characteristics.

Some implementations comprise the image data including first image data corresponding to a first optical sensor of the localizer camera and second image data corresponding to a second optical sensor of the localizer camera, each of the first and second image data indicating a blob for each active marker generated from a light signal emitted from the active marker, and identifying a first blob from the first image data and a second blob from the second image data that correspond to a same active marker; acquiring a first characteristic of the first blob and a second characteristic of the second blob; combining the acquired first characteristic and the acquired second characteristic to form a combined blob characteristic; comparing the combined blob characteristic to the optimal characteristic to determine if the combined blob characteristic is suboptimal; and responsive to determining that the combined blob characteristic is suboptimal based on the comparison, communicating a control signal to the tracker that causes the tracker to adjust the light signal emitted from the active marker corresponding to the first and second blobs.

Some implementations comprise the combined blob characteristic indicating a first value, the optimal characteristic indicating a second value, and comparing the first value to the second value, responsive to the comparison indicating that the first value is greater than the second value, communicating a control signal to the tracker that causes the tracker to reduce an intensity and/or duration of the light signal emitted from the active marker corresponding to the first and second blobs; and responsive to the comparison indicating that the first value is less than the second value, communicating a control signal to the tracker that causes the tracker to increase the intensity and/or duration of the light signal emitted from the active marker corresponding to the first and second blobs.

Some implementations comprise the acquired first and second characteristics being acquired intensity characteristics, and the optimal characteristic being an optimal blob intensity characteristic. Some implementations comprise the optimal blob intensity characteristic indicating an intensity value greater than or equal to 75% and less than or equal to 95% of a full scale intensity value of the localizer camera. Some implementations comprise the acquired first and second characteristics being acquired size characteristics, and the optimal characteristic being an optimal blob size characteristic. Some implementations comprise the acquired first and second characteristics being acquired shape characteristics, and the optimal characteristic being an optimal blob shape characteristic.

Some implementations comprise the combined blob characteristic being defined as a first combined blob characteristic, the optimal characteristic being defined as a first optimal characteristic, and acquiring a third characteristic of the first blob and a fourth characteristic of the second blob; combining the acquired third characteristic and the acquired fourth characteristic to form a second combined blob characteristic; comparing the second combined blob characteristic to a second optimal characteristic; and based on the comparison of the second combined blob characteristic to the second optimal characteristic, communicating a control signal to the tracker that causes the tracker to adjust the light signal emitted from the active marker corresponding to the first and second blobs. Some implementations comprise comparing the second combined blob characteristic to the second optimal characteristic to determine whether the second combined blob characteristic is suboptimal; and responsive to determining that the second combined blob characteristic is suboptimal based on the comparison, communicating the control signal the tracker that causes the tracker to adjust the light signal emitted from the active marker corresponding to the first and second blobs.

Some implementations comprise the combined blob characteristic being defined as a first combined blob characteristic, the optimal characteristic being defined as a first optimal characteristic, and comparing the first combined blob characteristic to the first optimal characteristic to determine whether the first combined blob characteristic is suboptimal; responsive to determining that the first combined blob characteristic is suboptimal, communicating the control signal to the tracker that causes the tracker to adjust the light signal emitted from the active marker corresponding to the first and second blobs; and responsive to determining that the first combined blob characteristic is not suboptimal based on the comparison: acquiring a third characteristic of the first blob and a fourth characteristic of the second blob; combining the acquired third characteristic and the acquired fourth characteristic to form a second combined blob characteristic; comparing the second combined blob characteristic to a second optimal characteristic to determine whether the second combined blob characteristic is suboptimal; and responsive to determining that the second combined blob characteristic is suboptimal based on the comparison, communicating a control signal to the tracker that causes the tracker to adjust the light signal emitted from the active marker corresponding to the first and second blobs.

Some implementations comprise the acquired first and second characteristics being blob intensity characteristics, and the acquired third and fourth characteristics being blob size characteristics or blob shape characteristics. Some implementations comprise the acquired first and second characteristics being blob size characteristics, and the acquired third and fourth characteristics being blob intensity characteristics or blob shape characteristics. Some implementations comprise the acquired first and second characteristics being blob shape characteristics, and the acquired third and fourth characteristics being blob intensity characteristics or blob size characteristics.

Some implementations comprise the object being defined as a first object, the blobs being defined as first blobs, the tracker being defined as a first tracker, the acquired characteristics being defined as acquired first characteristics, the optimal characteristic being defined as a first optimal characteristic specific to the first tracker, and a second tracker disposed relative to a second object in the surgical workspace and including a predefined geometry of active markers for tracking a pose of the second tracker in the surgical workspace, wherein the image data generated by the localizer camera includes a second blob for each of the active markers of the second tracker generated from a light signal emitted from the active marker of the second tracker. Some implementations further comprise assigning each of the second blobs to the active marker of the second tracker corresponding to the second blob; acquiring a second characteristic of each second blob; comparing the acquired second characteristics to a second optimal characteristic that is specific to the second tracker and differs from the first optimal characteristic; and based on the comparison, communicating at least one control signal to the second tracker that causes the second tracker to adjust the light signal emitted from at least one of the active markers of the second tracker.

Some implementations comprise, for each of the second blobs: comparing the acquired second characteristic of the second blob to the second optimal characteristic to determine whether the second blob is suboptimal; and responsive to determining that the second blob is suboptimal based on the comparison, communicating a control signal to the second tracker that causes the second tracker to adjust the light signal emitted from the active marker of the second tracker corresponding to the second blob.

Some implementations comprise assigning the first blobs to the active markers of the first tracker based on the first optimal characteristic. Some implementations comprise, for each of the first blobs: determining a difference between the acquired first characteristic of the first blob and the first optimal characteristic; determining whether the difference between the acquired first characteristic of the first blob and the first optimal characteristic is less than a threshold value; and responsive to determining that the difference between the acquired first characteristic of the first blob and the first optimal characteristic is less than the threshold value, determining that the first blob corresponds to the first tracker and assign the first blob to the active marker of the first tracker corresponding to the first blob.

Some implementations comprise assigning the second blobs to the active markers of the second tracker based on the second optimal characteristic. Some implementations comprise, for each of the second blobs: determining a difference between the acquired second characteristic of the second blob and the second optimal characteristic; determinizing whether the difference between the acquired second characteristic of the second blob and the second optimal characteristic is less than a threshold value; and responsive to determining that the difference between the acquired second characteristic of the second blob and the second optimal characteristic is less than the threshold value, determine that the second blob corresponds to the second tracker and assign the second blob the active marker of the second tracker corresponding to the second blob.+

Some implementations comprise the predefined geometry of active markers of the first tracker and the predefined geometry of active markers of the second tracker being substantially equivalent.

Some implementations comprise determining positions of the active markers of the tracker in the surgical workspace based on the image data; and based on the determined positions of the active markers, communicating the at least one control signal to the tracker that causes the tracker to adjust the light signal emitted from at least one of the active markers. Some implementations comprise, for each of the active markers, comparing the acquired characteristic of the blob corresponding to the active marker to the optimal characteristic to determine whether the blob corresponding to the active marker is suboptimal; and responsive to determining that the blob corresponding to the active marker is suboptimal, communicating a control signal to the tracker that causes the tracker to adjust the light signal emitted from the active marker based on the determined position of the active marker.

Some implementations comprise couniting a control signal to the tracker that causes the tracker to adjust the light signal emitted from the active marker based on the determined position of the active marker by comparing the determined position of the active marker to a previously determined position of the active marker to determine a change in distance between the active marker and the localizer camera; and based on the change in distance, communicating a control signal to the tracker that causes the tracker to adjust the light signal emitted from the active marker. Some implementations comprise communicating a control signal to the tracker that causes the tracker to adjust the light signal emitted from the active marker based on the determined change in distance by determining whether the change in distance indicates an increase or a decrease in the distance between the active marker and the localizer camera; responsive to the change in distance indicating an increase in the distance between the active marker and the localizer camera, communicate a control signal to the tracker that causes the tracker to increase an intensity and/or duration of the light signal emitted from the active marker; and responsive to the change in distance indicating a decrease in the distance between the active marker and the localizer camera, communicate a control signal to the tracker that causes the tracker to reduce an intensity and/or duration of the light signal emitted from the active marker.

Some implementations comprise the tracker including at least one actuator for repositioning the active markers of the tracker, and based on the comparison of the acquired characteristics to the optimal characteristic, communicating at least one control signal to the tracker that causes the tracker to reposition at least one of the active markers. Some implementations comprise, for each of the blobs, comparing the acquired characteristic of the blob to the optimal characteristic to determine whether the blob is suboptimal; and responsive to determining that the blob is suboptimal based on the comparison, communicating a control signal to the tracker that causes the tracker to reposition the active marker corresponding to the blob. Some implementations comprise the acquired characteristic of each blob indicating a first value, the optimal characteristic indicating a second value, and for each blob comparing the first value indicated for the blob to the second value; responsive to the comparison indicating that the first value for the blob is greater than the second value, communicating a control signal to the tracker that causes the tracker to reposition the active marker corresponding to the blob away from the localizer camera; and responsive to the comparison indicating that the first value for the blob is less than the second value, communicating a control signal to the tracker that causes the tracker to reposition the active marker corresponding to the blob towards from the localizer camera.

Some implementations comprise adjusting at least one optical parameter of the localizer camera based on the comparison by adjusting the light signal emitted from the light source to illuminate the passive markers based on the comparison. Some implementations comprise adjusting at least one optical parameter of the localizer camera based on the comparison by adjusting an intensity and/or duration of the light signal emitted from the light source to illuminate the passive markers based on the comparison.

Some implementations comprise combining the acquired characteristics to form a combined blob characteristic; comparing the combined blob characteristic to the optimal characteristic to determine whether the combined blob characteristic is suboptimal; and responsive to determining that the combined blob characteristic is suboptimal based on the comparison, adjusting the at least one optical parameter of the localizer camera. Some implementations comprise the combined blob characteristic indicating a first value, the optimal characteristic indicating a second value, and comparing the first value to the second value; responsive to the comparison indicating that the first value is greater than the second value, reducing an intensity and/or duration of the light signal emitted from the light source to illuminate the passive markers; and responsive to the comparison indicating that the first value is less than the second value, increasing the intensity and/or duration of the light signal emitted from the light source to illuminate the passive markers.

Some implementations comprise the acquired characteristics being defined as acquired first characteristics, the combined blob characteristic being defined as a first combined blob characteristic, the optimal characteristic being defined as a first optimal characteristic, and comparing the first combined blob characteristic to the first optimal characteristic to determine whether the first combined blob characteristic is suboptimal; responsive to determining that the first combined blob characteristic is suboptimal based on the comparison, adjusting the at least one optical parameter of the localizer camera; and responsive to determining that the first combined blob characteristic is not suboptimal based on the comparison: acquiring a second characteristic of each blob; combining the acquired second characteristics to form a second combined blob characteristic; comparing the second combined blob characteristic to a second optimal characteristic to determine whether the second combined blob characteristic is suboptimal; and responsive to determining that the second combined blob characteristic is suboptimal based on the comparison, adjust the at least one optical parameter of the localizer camera.

Some implementations comprise the object being defined as a first object, the blobs being defined as first blobs, the tracker being defined as a first tracker, the light signal being defined as a first light signal specific to the first tracker, and a second tracker disposed relative to a second object in the surgical workspace and including a predefined geometry of passive markers for tracking a pose of the second tracker in the surgical workspace. Some implementations further comprise emitting a second light signal specific to the second tracker from the light source, the second light signal having at least one characteristic that differs from at least one corresponding characteristic of the first light signal; receiving image data corresponding to the second light signal generated by the localizer camera, the received image data indicating a second blob for each of the passive markers of the second tracker generated from a reflection by the passive marker of the second light signal emitted from the light source; acquiring a characteristic of each second blob; comparing the acquired characteristics of the second blobs to the optimal characteristic to determine whether the acquired characteristics of the second blobs are suboptimal; and responsive to determining that the acquired characteristics of the second blobs are suboptimal based on the comparison, adjusting the at least one characteristic of the second light signal.

Some implementations comprise the at least one characteristic of the second light signal that differs from the at least one corresponding characteristic of the first light signal including a light intensity characteristic and/or light duration characteristic. Some implementations comprise the image data corresponding to the second light signal indicating a third blob for each of the passive markers of the first tracker generated from a reflection by the passive marker of the second light signal emitted from the light source, and responsive to receiving the image data corresponding to the second light signal, differentiating the second blobs from the third blobs based on the optimal characteristic. Some implementations comprise differentiating the second blobs from the third blobs based on the optimal characteristic by acquiring a characteristic of each third blob; comparing the acquired characteristics of the second and third blobs to the optimal characteristic; and differentiating the second blobs from the third blobs based on the comparison of the acquired characteristics of the second and third blobs to the optimal characteristic.

Some implementations comprise, for each of the second and third blobs, determining a difference between the acquired characteristic of the blob and the optimal characteristic; determining whether the difference is less than a threshold value; and responsive to determining that the difference is less than the threshold value, determining that the blob corresponds to one of the second blobs. Some implementations comprise the predefined geometry of passive markers of the first tracker and the predefined geometry of passive markers of the second tracker being substantially equivalent.

Some implementations comprise emitting light signals from the light source having varying characteristics; receiving image data generated by the localizer camera for each of the emitted light signals that indicates a blob for each of the passive markers generated from a reflection by the passive marker of the emitted light signal; for each instance of received image data, acquiring a characteristic of each blob indicated by the image data and comparing the acquired characteristics to the optimal characteristic to determine which of the instances of received image data is closest to optimal; responsive to determining the instance of received image data closest to optimal, assigning the characteristics of the light signal corresponding to the instance of received image data to the tracker; and tracking a pose of the tracker in the surgical workspace based on the light signal characteristics assigned to the tracker.

Some implementations comprise tracking a pose of the tracker in the surgical workspace based on the light signal characteristics assigned to the tracker by emitting a light signal from the light source having the light signal characteristics assigned to the tracker to illuminate the passive markers of the tracker; receiving image data generated by the localizer camera corresponding to the emitted light signal having the light signal characteristics assigned to the tracker; and determining a pose of the tracker in the surgical workspace based on the received image data. Some implementations comprise emitting a light signal from the light source having the light signal characteristics assigned to the tracker to illuminate the passive markers of the tracker; receiving image data corresponding to the emitted light signal having the light signal characteristics assigned to the tracker, the received image data indicating a blob for each passive marker of the tracker generated from a reflection of the emitted light signal having the light signal characteristics assigned to the tracker by the passive marker; acquiring a characteristic of each of the blobs in the received image data; comparing the acquired characteristics of the blobs in the received image data to the optimal characteristic to determine whether the acquired characteristics of the blobs are suboptimal; and responsive to determining that the acquired characteristics of the blobs are suboptimal based on the comparison, adjusting the light signal characteristics assigned to the tracker.

Some implementations comprise determining positions of the passive markers of the tracker in the surgical workspace based on the image data; and based on the determined positions of the passive markers, adjusting the at least one optical parameter of the localizer camera. Some implementations comprise comparing the acquired characteristics of the blobs to the optimal characteristic to determine whether the blobs are suboptimal; and responsive to determining that the blobs are suboptimal based on the comparison, adjusting the at least one optical parameter of the localizer camera based on the determined positions of the passive markers.

Some implementations comprise adjusting the at least one optical parameter of the localizer camera based on the determined positions of the passive markers by determining an average distance between the passive markers and the localizer camera based on the determined positions of the passive markers; comparing the determined average distance to a previously determined average distance between the passive markers and the localizer camera to determine a change in the average distance between the passive markers and the localizer camera; and based on the change in average distance, adjusting the at least one optical parameter of the localizer camera. Some implementations comprise adjusting the at least one optical parameter of the localizer camera based on the change in average distance by determining whether the change in average distance indicates an increase or a decrease in the average distance between the passive markers and the localizer camera; responsive to the change in distance indicating an increase in the average distance between the passive markers and the localizer camera, creasing an intensity and/or duration of the light signal emitted from the light source to illuminate the passive markers; and responsive to the change in distance indicating a decrease in the average distance between the passive marker and the localizer camera, reducing an intensity and/or duration of the light signal emitted from the light source to illuminate the passive markers.

Some implementations comprise the passive markers of the tracker being manually repositionable, and based on the comparison of the acquired characteristics to the optimal characteristic, determining and displaying guidance for repositioning the passive markers of the tracker. Some implementations comprise, for each of the blobs, assigning the blob to the passive marker corresponding to the blob; comparing the acquired characteristic of the blob to the optimal characteristic to determine whether the blob is suboptimal; and responsive to determining that the blob is suboptimal based on the comparison, determining and displaying guidance for repositioning the passive marker corresponding to the blob.

Some implementations comprise the acquired characteristic of each blob indicating a first value, the optimal characteristic indicating a second value, and for each blob, assigning the blob to the passive marker corresponding to the blob; comparing the first value indicated for the blob to the second value; responsive to the comparison indicating that the first value for the blob is greater than the second value, determining and displaying guidance to reposition the passive marker corresponding to the blob away from the localizer camera; and responsive to the comparison indicating that the first value for the blob is less than the second value, determining and displaying guidance to reposition the passive marker corresponding to the blob towards from the localizer camera 18.

Some implementations comprise adjusting the at least one optical parameter of the localizer camera based on the comparison by adjusting an electronic aperture time of the localizer camera. Some implementations comprise the optimal characteristic indicating a first value, and combining the acquired characteristics to form a combined blob characteristic indicating a second value; comparing the second value to the first value; responsive to the comparison indicating that the second value is greater than the first value, reducing the electronic aperture time of the localizer camera; and responsive to the comparison indicating that the second value is less than the first value, increasing the electronic aperture time of the localizer camera.

Some implementations comprise the localizer camera including a mechanical shutter, and adjusting the at least one optical parameter of the localizer camera based on the comparison by adjusting a shutter time of the mechanical shutter. Some implementations comprise the optimal characteristic indicating a first value, and combining the acquired characteristics to form a combined blob characteristic indicating a second value; comparing the second value to the first value; responsive to the comparison indicating that the second value is greater than the first value, reducing the shutter time of the mechanical shutter; and responsive to the comparison indicating that the second value is less than the first value, increasing the shutter time of the mechanical shutter.

Some implementations comprise the localizer camera including a mechanical aperture, and the adjusting the at least one optical parameter of the localizer camera based on the comparison by adjusting a capture size of the mechanical aperture. Some implementations comprise the optimal characteristic indicating a first value, and combining the acquired characteristics to form a combined blob characteristic indicating a second value; comparing the second value to the first value; responsive to the comparison indicating that the second value is greater than the first value, reducing the capture size of the mechanical aperture; and responsive to the comparison indicating that the second value is less than the first value, increasing the capture size of the mechanical aperture.

### BRIEF DESCRIPTION OF THE DRAWINGS

FIG. 1 illustrates a surgical system including a surgical navigation system for optimizing tracking of an object in a surgical workspace.
FIG. 2 illustrates components of the surgical system of FIG. 1.
FIG. 3 illustrates a method for optimizing tracking of an object in a surgical workspace using active trackers.
FIG. 4 illustrates image data that may be generated by a localizer camera of a surgical navigation system.
FIG. 5 illustrates trackers that may be affixed to objects in a surgical workspace for tracking such objects.
FIG. 6 illustrates suboptimal image data that may be generated by a localizer camera of a surgical navigation system.
FIG. 7 illustrates optimal image data that may be generated by a localizer camera of a surgical navigation system.
FIG. 8 illustrates a method for optimizing tracking of an object in a surgical workspace using passive trackers.
FIG. 9A illustrates an active tracker with a repositionable active marker oriented in a first direction.
FIG. 9B illustrates the active tracker of FIG. 9A with the repositionable active marker oriented in a second direction.
FIG. 10A illustrates a passive tracker with a repositionable passive marker oriented in a first direction.
FIG. 10B illustrates the passive tracker of FIG. 10A with the repositionable passive marker oriented in a second direction.

### DETAILED DESCRIPTION

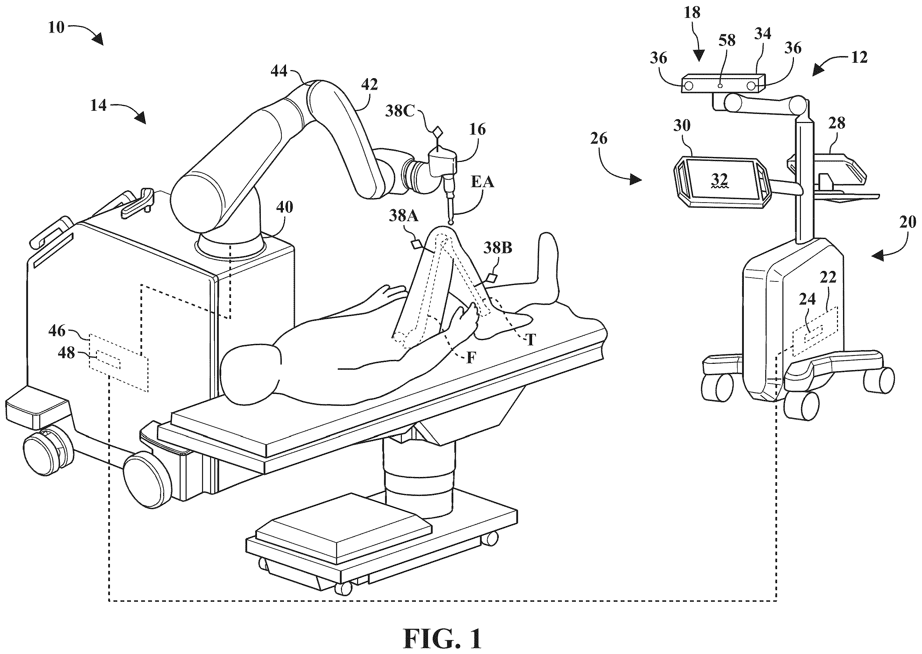

FIG. 1 illustrates a surgical system 10 for treating a patient. The surgical system 10 may be located in a surgical setting such as an operating room of a medical facility. The surgical system 10 may include a surgical navigation system 12 and a robotic manipulator 14. The robotic manipulator 14 may be coupled to a surgical instrument 16, and may be configured to maneuver the surgical instrument 16 to treat a target volume of patient tissue, such as at the direction of a surgeon and/or the surgical navigation system 12. For example, the surgical navigation system 12 may cause the robotic manipulator 14 to maneuver the surgical instrument 16 to remove the target volume of patient tissue while avoiding other objects adjacent the target volume in the surgical workspace, such as other medical tools and adjacent anatomical structures. Alternatively, the surgeon may manually hold and maneuver the surgical instrument 16 while receiving guidance from the surgical navigation system 12. As some non-limiting examples, the surgical instrument 16 may be a burring instrument, an electrosurgical instrument, an ultrasonic instrument, a reamer, an impactor, or a sagittal saw.

During a surgical procedure, the surgical navigation system 12 may be configured to track the pose (location and orientation) of objects of interest within the surgical workspace using tracker-based localization. The surgical workspace may include the target volume of patient tissue being treated and the areas surrounding the target volume in which an obstacle to treatment may be present. The tracked objects may include, but are not limited to, anatomical structures of the patient, surgical instruments such as the surgical instrument 16, and anatomical structures of surgical personnel such as the surgeon's hand or fingers. The tracked anatomical structures of the patient may include soft tissue such as ligaments, muscle, and skin, and may include hard tissue such as bone. The tracked surgical instruments may include retractors, cutting tools, and waste management devices used during the surgical procedure.

Each object of interest may be affixed to a tracker that is configured to transmit light signals to the surgical navigation system 12. The surgical navigation system 12 may be configured to detect such light signals by imaging the trackers, and to determine the poses of the trackers in the surgical workspace based on the imaging. The surgical navigation system 12 may then be configured to determine the poses of the objects in the surgical workspace based on the determined poses of the trackers and predetermined positional relationships between the objects and trackers.

The surgical navigation system 12 may also be configured to optimize the tracking of objects in the surgical workspace, such as by optimizing the light signals transmitted from the trackers to improve tracking precision. In particular, if the light signals transmitted from a tracker are suboptimal for the current position of the tracker relative to the imaging device of the surgical navigation system 12 and/or for the current ambient lighting conditions, then the navigation system 12 may have difficulty precisely tracking the tracker in the surgical workspace. For instance, if the intensities of the light signals are too low, then the navigation system 12 may detect an insufficient portion of the light signals. Alternatively, if the intensities of the light signals are too high, then the navigation system 12 may generate undesired artifacts when imaging the tracker. Either instance may impact the surgical navigation system's 12 ability to accurately pinpoint the position of the light signals transmitted from the tracker, which may correspondingly impact the tracking precision provided by the surgical navigation system 12. Accordingly, responsive to detecting a light signal from a tracker, the navigation system 12 may be configured to compare the detected light signal against optimal characteristics and to adjust the light signal transmitted from the tracker to obtain the optimal characteristics based on the comparison.

Responsive to determining the poses of objects of interest in the surgical workspace, the surgical navigation system 12 may display the relative poses of the tracked objects to aid the surgeon. The surgical navigation system 12 may also control and/or constrain movement of the robotic manipulator 14 and/or surgical instrument 16 based on virtual boundaries associated with the tracked objects. For example, the surgical navigation system 12 may identify a target volume of patient tissue to be treated and potential obstacles in the surgical workspace based on the tracked objects. The surgical navigation system 12 may then restrict a surgical tool (*e.g.,* an end effector EA of the surgical instrument 16) from contacting anything beyond the target volume of patient tissue to be treated, improving patient safety and surgical accuracy. The surgical navigation system 12 may also eliminate damage to surgical instruments caused by unintended contact with other objects, which may also result in undesired debris at the target site.

As illustrated in FIG. 1, the surgical navigation system 12 may include a localizer camera 18 and a navigation cart assembly 20. The navigation cart assembly 20 may house a navigation controller 22 configured to implement the functions, features, and processes of the surgical navigation system 12 described herein. In particular, the navigation controller 22 may include a processor 24 programmed to implement the functions, features, and processes of the navigation controller 22 and surgical navigation system 12 described herein. For instance, the processor 24 may be programmed to convert optical-based image data received from the localizer camera 18 into object pose data indicative of the poses of the tracked objects in the surgical workspace.

The navigation controller 22 may be in operative communication with a user interface 26 of the surgical navigation system 12. The user interface 26 may facilitate user interaction with the surgical navigation system 12 and navigation controller 22. For example, the user interface 26 may include one or more output devices that provide information to a user, such as from the navigation controller 22. The output devices may include a display 28 adapted to be situated outside of a sterile field including the surgical workspace and may include a display 30 adapted to be situated inside the sterile field. The displays 28, 30 may be adjustably mounted to the navigation cart assembly 20. The user interface 26 may also include one or more input devices that enable user-input to the surgical navigation system 12. The input devices may include a keyboard, mouse, and/or touch screen 32 that can be interacted with by a user to input surgical parameters to and control aspects of the navigation controller 22. The input devices may also include a microphone that enables user-input through voice-recognition technology.

The localizer camera 18 may be configured to facilitate the identification of the poses of the tracked objects in the surgical workspace by generating image data indicating the poses of trackers affixed to the objects. Specifically, the localizer camera 18 may be communicatively coupled to the navigation controller 22 of the surgical navigation system 12, and may be configured to generate and communicate the image data to the navigation controller 22 that indicates the poses of the trackers in the surgical workspace. The navigation controller 22 may then be configured to generate object pose data indicative of the poses of the objects affixed to the trackers in the surgical workspace based on the image data and predetermined positional relationships between the objects and trackers.

The localizer camera 18 may have an outer casing 34 that houses at least two optical sensors 36. Each of the optical sensors 36 may be adapted to detect light signals of a particular frequency band that are transmitted by the trackers, such as nonvisible light signals (*e.g.,* infrared or ultraviolet). While FIG. 1 illustrates the localizer camera 18 as a single unit with multiple optical sensors 36, in an alternative example, the localizer camera 18 may include separate units arranged around the surgical workspace, each with a separate outer casing 34 and one or more optical sensors 36.

The optical sensors 36 may be one-dimensional or two-dimensional charge-coupled devices (CCDs). For example, the outer casing 34 may house two two-dimensional CCDs for triangulating the position of trackers in the surgical workspace, or may house three one-dimensional CCDs for triangulating the position of the trackers in the surgical workspace. Additionally or alternatively, the localizer camera 18 may employ other optical sensing technologies, such as complementary metal-oxide semiconductor (CMOS) active pixels.

The localizer camera 18 may be mounted to an adjustable arm to selectively position the optical sensors 36 with a field of view of the surgical workspace and target volume that, ideally, is free from obstacles. The localizer camera 18 may be adjustable in at least one degree of freedom by rotating about a rotational joint, and may be adjustable about two or more degrees of freedom.

As previously described, the localizer camera 18 may cooperate with a plurality of trackers 38 to determine the position of objects within the surgical workspace to which the trackers 38 are affixed. In general, the object to which each tracker 38 is affixed may be rigid and inflexible so that movement of the object cannot or is unlikely to alter the positional relationship between the object and the tracker 38. In other words, the relationship between a tracker 38 in the surgical workspace and an object to which the tracker 38 is attached may remain fixed, notwithstanding changes in the position of the object within the surgical workspace. For instance, the trackers 38 may be firmly affixed to patient bones and surgical instruments, such as retractors and the surgical instrument 16. In this way, responsive to determining a position of a tracker 38 in the surgical workspace using the localizer camera 18, the navigation controller 22 may infer the position of the object to which the tracker 38 is affixed based on the determined position of the tracker.

For example, when the target volume to be treated is located at a patient's knee area, a tracker 38A may be firmly affixed to the femur F of the patient, a tracker 38B may be firmly affixed to the tibia T of the patient, and a tracker 38C may be firmly affixed to the surgical instrument 16. Trackers 38A, 38B may be attached to the femur F and tibia T in the manner shown in U.S. Patent No. 7,725,162, hereby incorporated by reference. Trackers 38A, 38B may also be mounted like those shown in U.S. Patent No. 9,566,120, hereby incorporated by reference. The tracker 38C may be integrated into the surgical instrument 16 during manufacture or may be separately mounted to the surgical instrument 16 in preparation for a surgical procedure.

Prior to the start of a surgical procedure using the surgical system 10, pre-operative images may be generated for anatomy of interest, such as anatomical structures defining and/or adjacent a target volume of patient tissue to be treated by the surgical instrument 16. For example, when the target volume of patient tissue to be treated is in the patient's knee area, pre-operative images of the patient's femur F and tibia T may be taken. These images may be based on MRI scans, radiological scans, or computed tomography (CT) scans of the patient's anatomy, and may be used to develop virtual models of the anatomical structures. Each virtual model for an anatomical structure may include a three-dimensional model (*e.g.,* point cloud, mesh, CAD) that includes data representing the entire or at least a portion of the anatomical structure, and/or data indicating a portion of the anatomical structure to be treated. These virtual models may be provided to and stored in the navigation controller 22 in advance of a surgical procedure.

In addition or alternatively to taking pre-operative images, plans for treatment can be developed in the operating room from kinematic studies, bone tracing, and other methods. These same methods may also be used to generate the virtual models described above.

In addition to virtual models corresponding to the patient's anatomical structures of interest, prior to the surgical procedure, the navigation controller 22 may receive and store virtual models for other tracked objects of interest, such as surgical instruments and other objects potentially present in the surgical workspace (*e.g.,* the surgeon's hand and/or fingers). The navigation controller 22 may also receive and store a virtual model for each tracker 38 disposed in the surgical workspace, and positional relationships between each tracker 38 and the object to which the tracker 38 is affixed. For instance, each positional relationship between a tracker 38 and the object to which the tracker 38 is affixed may be represented in the navigation controller 22 by a relationship model that combines the virtual model of the tracker 38 and the virtual model of the object in a common three-dimensional coordinate system. In this way, responsive to identifying the pose of the tracker 38 in the surgical workspace, the navigation controller 22 may reference the relationship model for the tracker 38 to determine the pose of the object to which the tracker 38 is affixed in the surgical workspace.

In some examples, the positional relationship between each tracker 38 and the object to which the tracker 38 is affixed may be indicated manually via the user interface 26. Alternatively, the positional relationship between each tracker 38 and the object to which the tracker 38 is affixed may be determined by tracing the object with a pointer instrument having its own fixed tracker 38 that is tracked by the navigation system 12 during the tracing, with the navigation system 12 also concurrently tracking the tracker 38 affixed to the object to correlate a pose of the traced object to a pose of the affixed tracker 38.

The navigation controller 22 may also receive and store surgical plan data prior to a procedure. The surgical plan data may identify the patient anatomical structures involved in the surgical procedure, may identify the instruments being used in the surgical procedure, and may define the planned trajectories of instruments and the planned movements of patient tissue during the surgical procedure.

During the surgical procedure, the optical sensors 36 of the localizer camera 18 may detect light signals, such as non-visible light signals (*e.g.,* infrared or ultraviolet), emitted from the trackers 38, and may output optical-based signals indicating the image plane positions in which the optical sensors 36 detected the light signals. The localizer camera 18 may be configured to consolidate these signals into image data that is then communicated to the navigation controller 22. The navigation controller 22 may be configured to generate object pose data indicating the positions of the objects to which the trackers 38 are affixed in a common coordinate system, such as a coordinate system specific to the localizer camera 18, based on the image data and the predefined positional relationships between the trackers 38 and objects.

The surgical instrument 16 may form part of an end effector of the robotic manipulator 14. The robotic manipulator 14 may include a base 40, several links 42 extending from the base 40, and several active joints 44 for moving the surgical instrument 16 with respect to the base 40. The links 42 may form a serial arm structure as shown in FIG. 1, a parallel arm structure, or other suitable structure. The robotic manipulator 14 may include an ability to operate in a manual mode in which a user grasps the end effector of the robotic manipulator 14 to cause movement of the surgical instrument 16 (*e.g.,* directly, or through force/torque sensor measurements that cause active driving of the robotic manipulator 14). The robotic manipulator 14 may also include a semi-autonomous mode in which the surgical instrument 16 is moved by the robotic manipulator 14 along a predefined tool path (*e.g.,* the active joints 44 of the robotic manipulator 14 are operated to move the surgical instrument 16 without requiring force/torque on the end effector from the user). An example of operation in a semi-autonomous mode is described in U.S. Pat. No. 9,119,655 to Bowling, et al., hereby incorporated by reference. A separate tracker 38 may be attached to the base 40 of the robotic manipulator 14 to also track movement of the base 40 by the localizer camera 18.

Similar to the surgical navigation system 12, the robotic manipulator 14 may house a manipulator controller 46 including a processor 48 programmed to implement the functions, features, and processes of the robotic manipulator 14, or more particularly of the manipulator controller 46, described herein. For example, the processor 48 may be programmed to control operation and movement of the surgical instrument 16 through movement of the links 42, such as at the direction of the surgical navigation system 12.

During a surgical procedure, the manipulator controller 46 may be configured to determine a desired location to which the surgical instrument 16 should be moved, such as based on navigation data received from the navigation controller 22. Based on this determination, and information relating to the current position of the surgical instrument 16, the manipulator controller 46 may be configured to determine an extent to which the links 42 need to be moved to reposition the surgical instrument 16 from the current position to the desired position. Data indicating where the links 42 are to be repositioned may be forwarded to joint motor controllers (*e.g.,* one for controlling each motor) that control the active joints 44 of the robotic manipulator 14. Responsive to receiving such data, the joint motor controllers may be configured to move the links 42 in accordance with the data, and consequently move the surgical instrument 16 to the desired position.

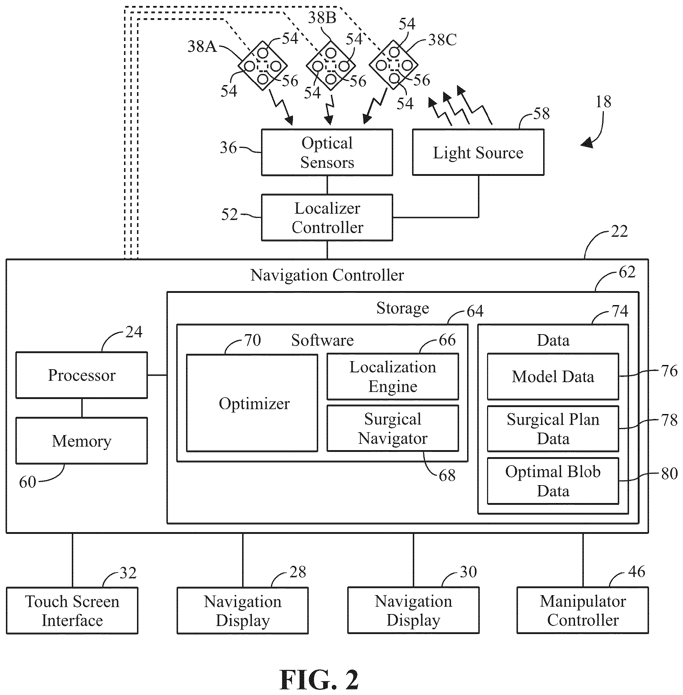

Referring now to FIG. 2, the localizer camera 18 may include a localizer controller 52 communicatively coupled to the optical sensors 36 and to the navigation controller 22. During a surgical procedure, the localizer controller 52 may be configured to operate the optical sensors 36 to cause them to generate optical-based signals indicative of detected light signals received from the trackers 38, or more particularly indicative of the image plane positions of the optical sensors 36 in which such light signals were detected.

The trackers 38 may each include a predefined geometry of markers 54 that direct light signals to the optical sensors 36. In some implementations, the trackers 38 may be active trackers 38, each having at least three active markers 54 that receive an electrical current from a power source to generate and emit light signals to the optical sensors 36. In this case, the trackers 38 may each be powered by an internal battery, or may have leads to receive power through the navigation controller 22. For instance, the active markers 54 may be light emitting diodes (LEDs) that transmit light, such as nonvisible light (*e.g.,* infrared or ultraviolet light), towards the optical sensors 36.

Each active tracker 38 may also include a tracker controller 56 communicatively coupled to the active markers 54 and to the navigation controller 22. The tracker controller 56 may be configured to control the rate and order in which the active markers 54 fire, such as at the direction of the navigation controller 22. For example, the tracker controllers 56 of the trackers 38 may cause the active markers 54 of each tracker 38 to fire at different rates and/or times to facilitate differentiation of the trackers 38 and/or markers 54 by the navigation controller 22. In some examples, the navigation controller 22 may form a bi-directional infrared communication channel with each tracker controller 56 to control the timing of the firing of the active markers 54, write/read nonvolatile data, and get the status (*e.g.,* battery level, broken LEDs) of the active tracker 38 or the object to which the active tracker 38 is affixed.

The sampling rate of the optical sensors 36 is the rate at which the optical sensors 36 detect light signals from sequentially fired markers 54. The optical sensors 36 may have sampling rates of 100 Hz or more, or more preferably 300 Hz or more, or most preferably 500 Hz or more. In one instance, the optical sensors 36 may have sampling rates of 8000 Hz.

Rather than being active, the trackers 38 may be passive trackers 38 including passive markers 54, such as reflectors that reflect light emitted from the localizer camera 18. Specifically, the localizer camera 18 may include a light source 58 that illuminates the trackers 38 with light, such as nonvisible light (*e.g.,* infrared or ultraviolet). The markers 54 may be configured to reflect the light back towards the localizer camera 18, which may then be detected by the optical sensors 36. In some instances, the surgical workspace may include a combination of active and passive trackers 38 for tracking various objects in the surgical workspace.

Responsive to the optical sensors 36 receiving light signals from the trackers 38, the optical sensors 36 may output optical-based signals to the localizer controller 52 indicating the poses of the trackers 38 relative to the localizer camera 18, and correspondingly, indicating the poses of the objects affixed to the trackers 38 relative to the localizer camera 18. In particular, each optical sensor 36 may include a one- or two-dimensional sensor area (also referred to as an "image plane") that detects light signals from the trackers 38, and responsively outputs optical-based signals indicating pixel coordinates within the sensor area that each light signal was detected. The optical-based signals output from each optical sensor 36 may thus represent an image of the trackers 38 generated by the optical sensor 36 from the detected light signals, with the image including blobs in pixel coordinates corresponding to the positions in the image plane of the optical sensor 36 that light signals were detected. The detected position of each light signal may be based on the angle at which the light signal is received by the optical sensor 36, and may thus correspond to the position of the marker 54 in the surgical workspace that emitted the detected light signal towards the optical sensor 36.

The optical sensors 36 may communicate the optical-based signals to the localizer controller 52, which in turn may generate image data for each optical sensor 36 based on the optical-based signals received from the optical sensor 36 and communicate such image data to the navigation controller 22. The image data for an optical sensor 36 may indicate the image and/or image plane positions represented by the optical-based signals received from the optical sensor 36. The navigation controller 22 may then generate tracker pose data indicating the poses of the trackers 38 relative to the localizer camera 18 based on the received image data. More particularly, the navigation controller 22 may determine a position of the trackers 38 in a coordinate system of the localizer camera 18 based on the image data. For instance, the navigation controller 22 may be configured to correlate blobs corresponding to a same marker 54 in image data concurrently generated for each optical sensor 36, triangulate the positions of the markers 54 relative to the localizer camera 18 based on the positions of the correlated blobs in the image data and a predetermined positional relationship between the optical sensors 36, and assign the triangulated positions to the predefined geometries of the markers 54 of each tracker 38 to determine the pose of each tracker 38 relative to the localizer camera 18.

Thereafter, the navigation controller 22 may generate object pose data indicating the poses of the objects affixed to the trackers 38 relative to the localizer camera 18 based on the tracker pose data. Specifically, the navigation controller 22 may retrieve the stored positional relationships between the trackers 38 and the objects to which the trackers 38 are affixed, and may apply these positional relationships to the tracker pose data to determine the poses of the objects fixed to the trackers 38 relative to the localizer camera 18. Alternatively, the localizer controller 52 may be configured to determine the tracker pose data and/or object pose data based on the optical-based signals generated by the optical sensors 36, and to transmit the tracker pose data and/or object pose data to the navigation controller 22 for further processing.

As previously described, the navigation controller 22 may include a processor 24 programmed to perform the functions, features, and processes of the navigation controller 22 described herein. The navigation controller 22 may also include memory 60 and non-volatile storage 62 each operatively coupled to the processor 24.

The processor 24 may include one or more devices selected from microprocessors, micro-controllers, digital signal processors, microcomputers, central processing units, field programmable gate arrays, programmable logic devices, state machines, logic circuits, analog circuits, digital circuits, or any other devices that manipulate signals (analog or digital) based on operational instructions stored in the memory 60. The memory 60 may include a single memory device or a plurality of memory devices including, but not limited to, read-only memory (ROM), random access memory (RAM), volatile memory, non-volatile memory, static random access memory (SRAM), dynamic random access memory (DRAM), flash memory, cache memory, or any other device capable of storing information. The non-volatile storage 62 may include one or more persistent data storage devices such as a hard drive, optical drive, tape drive, non-volatile solid state device, or any other device capable of persistently storing information.

The non-volatile storage 62 may store software 64, which may include one or more applications and/or modules such as a localization engine 66, a surgical navigator 68, and an optimizer 70. Each application or module may be embodied by a distinct set of computer-executable instructions compiled or interpreted from a variety of programming languages and/or technologies, including, without limitation, and either alone or in combination, Java, C, C++, C#, Objective C, Fortran, Pascal, Java Script, Python, Perl, and PL/SQL. The processor 24 may operate under control of the software 64 stored in the non-volatile storage 62. In particular, the processor 24 may be configured to read into the memory 60 and execute the computer-executable instructions embodying the software 64. Upon execution by the processor 24, the computer-executable instructions may be configured to cause the processor 24 to implement the configured functions, features, and processes of the navigation controller 22 described herein.

The non-volatile storage 62 of the navigation controller 22 may also store data 74 that facilitates operation of the navigation controller 22. Specifically, the software 64 of the navigation controller 22 may be configured upon execution to access the data 74 to facilitate implementation of the functions, features, and processes of the navigation controller 22 described herein. For example, the data 74 stored in the non-volatile storage 62 may include model data 76, surgical plan data 78, and optimal blob data 80.

The model data 76 may include the virtual models of the anatomical structures of interest to the surgical procedure, including the virtual models for potential obstacles such as a surgeon's hand or fingers, and the virtual models for the surgical instruments being used in the surgical procedure, as described above. The model data 76 may also include the virtual model for each tracker 38 that indicates the predetermined geometry of markers 54 of the tracker 38, and the positional relationships between each tracker 38 and the object to which the tracker 38 is affixed. The model data 76 may also indicate configuration parameters of the localizer camera 18, such as the positions of the optical sensors 36 in a coordinate system specific to the localizer camera 18, to enable triangulating the positions of the markers 54 in the coordinate system specific to the localizer camera 18 based on the image data generated by the localizer camera 18.

The surgical plan data 78 may identify patient anatomical structures and target volumes involved in the surgical procedure, may identify the instruments being used in the surgical procedure, and may define the planned trajectories of instruments and the planned movements of patient tissue during the surgical procedure. The optimal blob data 80 may indicate optimal characteristics for the blobs generated by the localizer camera 18 from light signals received from the markers 54 of the trackers 38 for optimizing the received light signals and improving tracking precision.

Referring again to the software 64 executable by the processor 24 of the navigation controller 22, the localization engine 66 may be configured to generate the tracker pose data indicative of the poses of the trackers 38 relative to the localizer camera 18, such as based on the image data received from the localizer camera 18. The localization engine 66 may also be configured to transform the pose of a tracker 38 relative to the localizer camera 18 to a pose of the object affixed to the tracker 38 relative to the localizer camera 18, such as based on the tracker pose data and the positional relationships indicated in the model data 76.

The surgical navigator 68 may be configured to provide surgical guidance based on the object pose data and the surgical plan data 78. For instance, the surgical navigator 68 may be configured to display the relative poses of the tracked objects on the navigation displays 28, 30, and may be configured to issue control commands to the robotic manipulator 14 to move the surgical instrument 16 while avoiding undesired contact with other tracked objects.

The optimizer 70 may be configured to optimize the tracking of objects in the surgical workspace, such as by adjusting the light signals transmitted by the markers 54 of the tracker 38 to the localizer camera 18 based on a comparison of the image data generated by the localizer camera 18 and the optimal blob data 80. Examples of such optimization are described in more detail below.

Each of the manipulator controller 46 and the localizer controller 52 may also include a processor, memory, and non-volatile storage including data and software configured, upon execution by the processor, to implement the functions, features, and processes of the controller described herein.

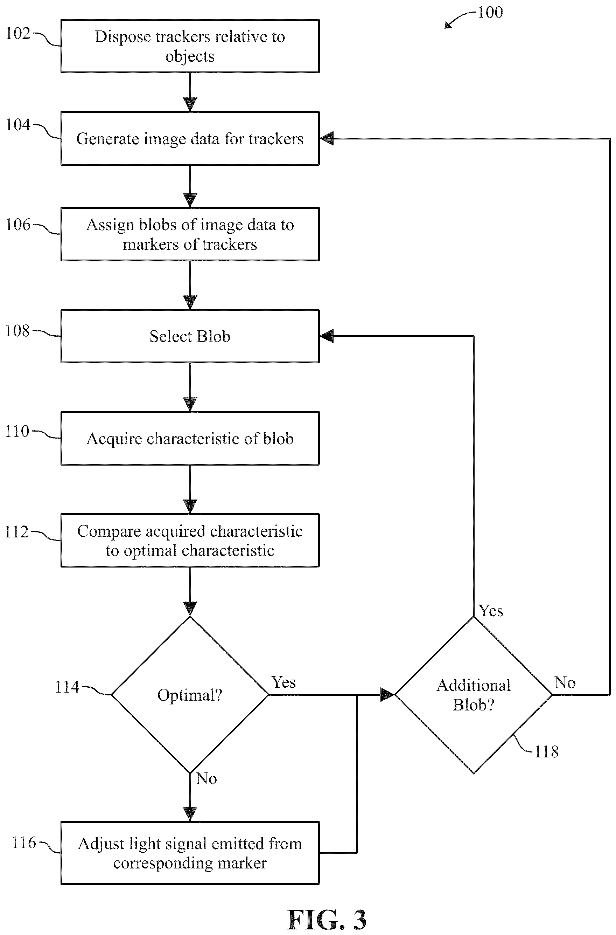

FIG. 3 illustrates a method 100 for optimizing the tracking of objects in a surgical workspace by adjusting the light signals emitted from the trackers 38 to improve tracking precision. The method 100 may be utilized when active trackers 38 including active markers 54 are present in the surgical workspace. The method 100 may be facilitated by the surgical navigation system 12, or more particularly by the navigation controller 22, such as upon execution of the software 64.

In block 102, trackers 38 may be disposed relative to objects in the surgical workspace desired to be tracked. In particular, a tracker 38 may be affixed to each object, with each tracker 38 including a predefined geometry of active markers 54. The positional relationship between each tracker 38, or more particularly the markers 54 of each tracker 38, and the object to which the tracker 38 is affixed may be stored as model data 76 in the non-volatile storage 62 of the navigation controller 22.

In block 104, image data may be generated by the localizer camera 18, such as at the direction of the navigation controller 22. In particular, the navigation controller 22 may communicate control signals to the tracker controllers 56 of the trackers 38 that instruct the tracker controllers 56 to fire light signals, such as nonvisible light signals, from the active markers 54. Contemporaneously, the navigation controller 22 may communicate a control signal to the localizer controller 52 that instructs the localizer controller 52 to operate the optical sensors 36 to detect the light signals emitted from the active markers 54. Each of the optical sensors 36 may responsively generate optical-based signals that indicate a blob for each active marker 54, with the blob having pixel coordinates corresponding to the position in the image plane of the optical sensor 36 that a light signal was received from the active marker 54. The localizer controller 52 may receive the optical-based signals from the optical sensors 36, and communicate image data corresponding to the optical-based signals to the navigation controller 22 as described above.

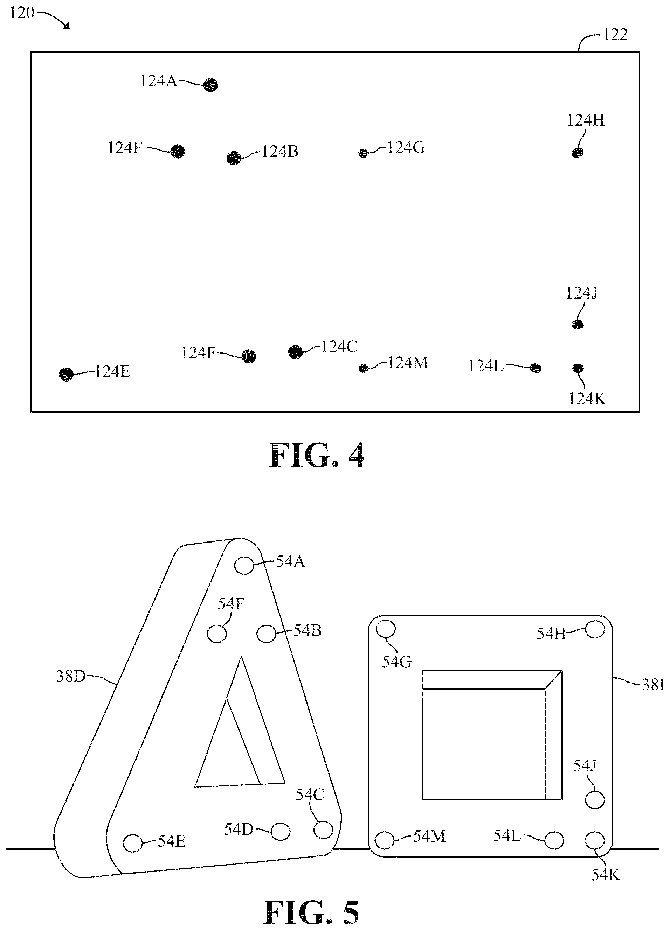

FIG. 4 illustrates image data 120 that may be generated for a two-dimensional optical sensor 36 of the localizer camera 18 from light signals emitted by the active markers 54 of the exemplary trackers 38 illustrated in FIG. 5. As shown in the illustrated example, the image data 120 may indicate a two-dimensional image 122 including blobs 124. Each of the blobs 124 may be generated from a light signal emitted from a different one of the active markers 54 of the trackers 38 illustrated in FIG. 5, and the pixel coordinates of each blob 124 in the image 122 may correspond to the position on the image plane of the optical sensor 36 in which the light signal corresponding to the blob 124 was detected. For instance, blob 124 may be generated from a light signal emitted from active marker 54A, blob 124B may be generated from a light signal emitted from active marker 54B, and so on.

Referring again to FIG. 3, in block 106, each blob 124 of the image data generated by the localizer camera 18 may be assigned to the active marker 54 of the trackers 38 corresponding to the blob 124, such as by the navigation controller 22 upon execution of the localization engine 66. For instance, the tracker controllers 56 of the trackers 38 may be configured to fire the active markers 54 at different times and/or rates, such as at the direction of the navigation controller 22, and the localizer camera 18 may be configured to generate distinct image data for each fired active marker 54. The navigation controller 22 may thus be able to correlate a blob 124 of each instance of received image data to the active marker 54 being fired when the image data was generated.

As a further example, such as if the active markers 54 are fired at the same time, the navigation controller 22 may be configured to correlate blobs 124 corresponding to a same marker 54 in image data concurrently generated for each optical sensor 36, such as by applying epipolar geometry to the image data based on the positional relationship between the optical sensors 36, which may be determined in advance and stored as model data 76 in the non-volatile storage 62 of the navigation controller 22. Thereafter, the navigation controller 22 may be configured to triangulate a three-dimensional position for each group of correlated blobs 124 relative to the localizer camera 18. The navigation controller 22 may then be configured to apply the model data 76 indicating the predetermined geometry of markers 54 of each tracker 38 to the triangulated positions to identify a triangulated position corresponding each marker 54 of the tracker 38, and assign the blobs accordingly.

For instance, assuming a tracker 38 with a predefined geometry of six markers 54 is present in the surgical workspace, the navigation controller 22 may be configured to identify each possible combination of six triangulated positions. For each possible combination, the navigation controller 22 may then be configured to determine whether the geometry formed by the triangulated positions of the combination corresponds to the predefined geometry of markers 54 of the tracker 38. If so, then the navigation controller 22 may be configured to assign each blob used to generate the triangulated positions of the combination to the marker 54 of the tracker 38 that generated the blob, such as by matching the relationship between the triangulated position corresponding to the blob and the other triangulated positions of the combination to one of the markers 54 of the predefined geometry.

As previously described, responsive to assigning blobs to the markers 54 of the tracker 38 that generated the blobs, the navigation controller 22 may be configured to determine a pose of the object to which the tracker 38 is affixed. Specifically, if not already calculated, the navigation controller 22 may be configured to triangulate the position of each marker 54 of the tracker 38 relative to the localizer camera 18 based on the positions of the blobs assigned to the marker 54 within the image data and the predetermined positional relationship between the optical sensors 36. The positions of the markers 54 relative to the localizer camera 18 indicate the pose of the tracker 38 relative to the localizer camera 18, and the navigation controller 22 may be configured to then determine a pose of the object to which the tracker 38 is affixed relative to the localizer camera 18 based on the triangulated positions of the markers 54 and the predetermined positional relationship between the tracker 38 and object, as described above.

The following blocks of the method 100 may concern optimizing the light signals emitted from the active markers 54 to improve tracking precision. In particular, emitting suboptimal light signals from the active markers 54 may result in suboptimal blobs being generated by the optical sensors 36, which in turn may lead to suboptimal or imprecise tracking. For instance, if the intensity of a light signal emitted from an active marker 54 is too low for the current ambient lighting conditions and the current distance between the active marker 54 and the localizer camera 18, then the localizer camera 18 may not adequately detect the light signal for the purposes of tracking the active marker 54. Alternatively, if the intensity of a light signal emitted from an active marker 54 is too high, then the light signal may oversaturate one or more pixels of the image plane of each optical sensor 36, which may introduce undesired artifacts in the image data that impacts the navigation controller's 22 ability to precisely track the active marker 54.

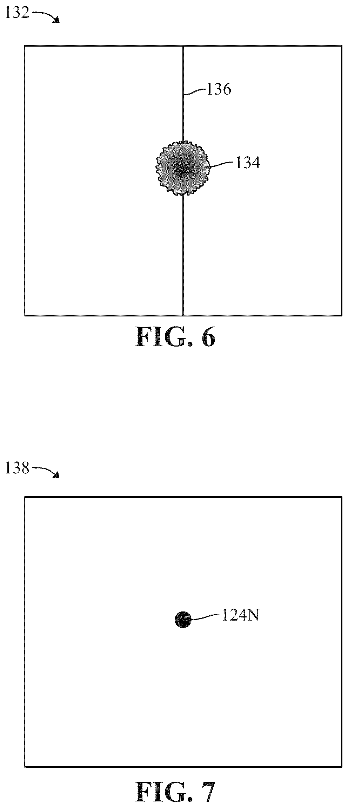

As an example, FIG. 6 illustrates exemplary image data 132 that may be generated by an optical sensor 36 from a light signal emitted from an active marker 54 that causes oversaturation of one or more pixels of the optical sensor 36. As shown in the illustrated example, the image data 132 may include undesired artifacts caused by the oversaturation, such as a blooming artifact 134 and a smear artifact 136. Such artifacts may cause the navigation controller 22 to imprecisely calculate the three-dimensional position of the active marker 54 relative to the localizer camera 18, which in turn may lead to imprecise tracking of the object to which the active marker 54 corresponds. Conversely, FIG. 7 illustrates exemplary image data 138 that may be generated by an optical sensor 36 from an optimal light signal emitted from an active marker 54. As shown in the illustrated example, the image data 138 may depict a blob 124N generated from the light signal that is circular and of uniform intensity.

Referring again to FIG. 3, in block 108, one of the blobs 124 of the image data may be selected, and in block 110, one or more characteristics of the selected blob 124 may be acquired. For instance, the navigation controller 22, such as via the optimizer 70, may identify an intensity characteristic, and/or a size characteristic, and/or a shape characteristic of the selected blob 124. The intensity characteristic may correspond to the magnitude of the light signal received by the optical sensor 36 that corresponds to the selected blob 124, and may be determined as the highest pixel intensity of the blob 124, an average pixel intensity of the blob 124, or a first moment of the blob 124. The size characteristic may correspond to the area of the blob 124 and may be determined by counting the number pixels forming the blob 124. The shape characteristic of the selected blob 124 may correspond to the perimeter of the selected blob 124 and may be determined using edge detection algorithms.

In block 112, the acquired characteristics may be compared with corresponding optimal characteristics, and in block 114, a determination may be made of whether the blob is optimal based on the comparison. More particularly, the non-volatile storage 62 of the navigation controller 22 may store optimal blob data 80 indicating one or more optimal blob characteristics. The optimal blob characteristics indicated by the optical blob data 80 may correspond to characteristics of a blob that enables the surgical navigation system 12 to accurately localize the marker 54 that generated the blob, and may thus be compared with the acquired characteristics to determine whether the blob is optimal for navigation purposes. For instance, the optimal blob data 80 may indicate an optimal intensity characteristic for comparison with the acquired intensity characteristic, and/or an optimal size characteristic for comparison with the acquired size characteristic, and/or an optimal shape characteristic for comparison with the acquired shape characteristic.

Each optimal blob characteristic may indicate an optimal value or a range of optimal values for which a corresponding acquired blob characteristic may be considered optimal. For example and without limitation, the optimal intensity characteristic may indicate a single intensity value that is greater than or equal to 75% and less than or equal to 95% of a full scale intensity value of the pixels of the optical sensor 36, such as 80%, 85%, or 90%. The full scale intensity value of the pixels of the optical sensor 36 may correspond to the maximum light intensity a given pixel can accommodate before becoming oversaturated. If the acquired intensity characteristic is greater than or less than the indicated optimal intensity value, then the acquired intensity characteristic may not be considered optimal.

Alternatively, the optimal intensity characteristic may indicate a range of optimal values defined by a lower intensity threshold value, such as 75% of the full scale intensity value of the pixels of the optical sensor 36, and an upper intensity threshold value, such as 95% of the full scale intensity of the pixels of the optical sensor 36. In this case, if the acquired intensity characteristic is greater than or equal to the lower threshold intensity value and less than or equal to the upper threshold intensity value, the acquired intensity characteristic may be considered optimal. As alternative non-limiting examples, the optimal intensity characteristic may indicate a range of 60% to 95%, 80% to 95%, or 85% to 95% of the full scale intensity value of the pixels of the optical sensor 36. The optimal size characteristic may similarly indicate an area value or a range of area values for which the acquired size characteristic may be considered optimal.

The optimal shape characteristic may indicate an optimal shape (*e.g.,* circle) with an optimal area, and may indicate an optimal ratio value (*e.g.,* one) or a range of optimal ratio values defined by a lower ratio threshold value (*e.g.,* 0.8) and an upper ratio threshold value *(e.g.,* 1.2). To compare the acquired shape characteristic of a given blob to the optimal shape characteristic, the navigation controller 22 may be configured to align the acquired shape of the blob with the optimal shape of the optimal shape characteristic, and to calculate the ratio of the area of the acquired shape that extends outside the optimal shape to the area of the optimal shape that extends outside the acquired shape. This calculated ratio may be considered to at least partly define the acquired shape characteristic of the given blob. If the optimal shape characteristic indicates a single optimal ratio value, then the acquired shape characteristic may be considered optimal if the calculated ratio is equal to the optimal ratio value. Alternatively, if the optimal shape characteristic indicates a range of optimal ratio values, then the acquired shape characteristic may be considered optimal if the calculated ratio is greater than or equal to the lower ratio threshold value and less than or equal to the upper ratio threshold value.

Responsive to determining that an acquired blob characteristic is suboptimal ("No" branch of block 114), in block 116, the light signal emitted from the active marker 54 corresponding to the blob 124 may be adjusted for future tracking of the marker 54, such as to cause the active marker 54 to emit a light signal that results in generation of a blob characteristic that is optimal or closer to optimal in future tracking. More particularly, the intensity and/or duration of the light signal emitted from the active marker 54 may be adjusted. For instance, the navigation controller 22, such as via the optimizer 70, may be configured to communicate a control signal to the tracker controller 56 for the active marker 54 that causes the tracker controller 56 to adjust the intensity and/or duration of the light signal emitted from the active marker 54 for future tracking of the marker 54. More specifically, if the acquired blob characteristic is greater than the one or more optimal values defined by the corresponding optimal blob characteristic, then the navigation controller 22 may be configured to communicate a control signal to the tracker controller 56 that causes the tracker controller 56 to decrease the intensity and/or duration of the light signal emitted from the active marker 54. Alternatively, if the acquired blob characteristic is less than the one or more optimal values defined by the corresponding optimal blob characteristic, then the navigation controller 22 may be configured to communicate a control signal to the tracker controller 56 that causes the tracker controller 56 to increase the intensity and/or duration of the light signal emitted from the active marker 54.

The intensity of the light signal emitted from an active marker 54 may be proportional to a magnitude of the current applied to the active marker 54. Accordingly, if the intensity of the light signal emitted from the active marker 54 is to be increased, then the control signal communicated to the tracker controller 56 may cause the tracker controller 56 to increase the current applied to the active marker 54 in future tracking iterations. Conversely, if the intensity of the light signal emitted from the active marker 54 is to be decreased, then the control signal communicated to the tracker controller 56 may cause the tracker controller 56 to decrease the current applied to the active marker 54 in future tracking iterations. The duration of the light signal emitted from an active marker 54 may be proportional to the duration in which current is applied to the active marker 54, which may be similarly adjusted to cause a shorter or greater duration.

The extent to which the intensity and/or duration of the emitted light signal is increased or reduced may be proportional to the difference between the acquired characteristic and the optimal characteristic. In addition or alternatively, the navigation controller 22 may be configured to implement a PID loop and/or stored lookup tables to determine an extent by which to increase or reduce the intensity and/or duration of the emitted light signal so as to make the acquired blob characteristic optimal.

In some examples, the navigation controller 22 may be configured to prioritize optimizing certain types of acquired blob characteristics over others. For instance, for a given blob 124, the navigation controller 22 may be configured to initially optimize an acquired intensity characteristic of the blob 124. Responsive to the acquired intensity characteristic becoming optimized, the navigation controller 22 may be configured to then optimize the acquired size characteristic. Responsive to the acquired size characteristic becoming optimized, the navigation controller 22 may be configured to then optimize the acquired shape characteristic. During each tracking and optimization iteration, the navigation controller 22 may thus be configured to acquire and check whether a type of blob characteristic of highest priority is optimal. If not, then the navigation controller 22 may be configured to adjust the light signal emitted from the corresponding active marker 54 to optimize the type of blob characteristic for future iterations, as described above. If the type of blob characteristic of highest priority is determined optimal, then the navigation controller 22 may be configured to acquire and check whether the type of blob characteristic of the next highest priority is optimal, and so on.

Responsive to determining that each of the acquired blob characteristics is optimal ("Yes" branch of block 114), or to adjusting the light signal emitted from the corresponding active marker 54 in block 116, in block 118, a determination may be made of whether the image data contains an additional blob 124 not yet checked against the optimal blob characteristics. If so ("Yes" branch of block 118), then the method 100 may return to block 108 to select the additional blob 124 and repeat blocks 110 through 116, if appropriate. If not ("No" branch of block 118), then the method 100 may return to block 104 to generate further image data for the trackers 38 in the surgical workspace, and so on. The light signal emitted from a given marker 54 may thus vary over time, and may be adjusted multiple times over a given surgical procedure.

In some instances, rather than optimizing each blob 124 separately, the navigation controller 22 may be configured to optimize blobs 124 of the image data that correspond to a same active marker 54 together. As previously described, the image data generated by the localizer camera 18 may include image data for each optical sensor 36, with each instance of image data indicating a blob for each active marker 54 in the surgical workspace emitting a light signal when the image data is captured. For each set of blobs within the image data corresponding to a same active marker 54, which may be determined as described above, the navigation controller 22 may be configured to acquire at least one characteristic of each blob. The navigation controller 22 may then be configured to combine the acquired characteristics of the same type (*e.g.,* intensity, size, shape) to form a combined blob characteristic of the type for the set of blobs, such as by averaging the value indicated by the acquired characteristics of the type. For instance, the navigation controller 22 may be configured to determine a combined blob intensity characteristic for a set of corresponding blobs 124 by averaging intensity values of acquired intensity characteristics of the corresponding blobs 124, determine a combined blob size characteristic for a set of corresponding blobs 124 by averaging areas indicated by acquired size characteristics of the corresponding blobs 124, and determine a combined blob shape characteristic for a set of corresponding blobs 124 by averaging the ratios indicated by acquired shape characteristics of the corresponding blobs 124.

The navigation controller 22 may then be configured to compare each combined blob characteristic to the corresponding optimal blob characteristic to determine if the combined blob characteristic is suboptimal. If so, then the navigation controller 22 may be configured to communicate a control signal to the tracker 38 including the active marker 54 corresponding to the combined blob characteristic that causes the tracker 38 to adjust the light signal emitted from the active marker 54, as described above.

To this end, the navigation controller 22 may also be configured to prioritize optimizing combined blob characteristics of certain types as described above. For instance, for a set of blobs 124 corresponding to a same active marker 54, the navigation controller 22 may be configured to initially determine a combined blob characteristic of a type that is of a highest priority (*e.g.,* blob intensity), and to compare the combined blob characteristic to the corresponding optimal characteristic to determine whether the combined blob characteristic is suboptimal. Responsive to determining that the combined blob characteristic of the highest priority type is suboptimal based on the comparison, the navigation controller 22 may be configured to communicate a control signal to the tracker 38 that causes the tracker 38 to adjust the light signal emitted from the active marker 54 corresponding to the combined blob characteristic, as described above.

Conversely, responsive to determining that the combined blob characteristic is not suboptimal based on the comparison, the navigation controller 22 may be configured to acquire characteristics of each blob in the set that are of a type of a next highest priority (*e.g.,* size, shape), combine these acquired characteristics to form a further combined blob characteristic of the type of the next highest priority, and compare the further combined blob characteristic to the optimal characteristic corresponding to the type of the next highest priority to determine whether the further combined blob characteristic is suboptimal. Responsive to determining that the further combined blob characteristic is suboptimal based on the comparison, the navigation controller 22 may be configured to communicate a control signal to the tracker that causes the tracker 38 to adjust the light signal emitted from the active marker 54 corresponding to the set of corresponding blobs 124, as described above.

In some alternative examples, the navigation controller 22 may be configured to optimize the light signal emitted from each active marker 54 based on blob characteristics acquired from only one of the blobs 124 corresponding to the active marker 54, such as the blob 124 indicated in the image data generated by a specified one of the optical sensors 36.

In some instances, different trackers 38 may be optimized to different optimal blob characteristics. To this end, the optimal blob data 80 may indicate different sets of one or more optimal blob characteristics for different trackers 38. For example, the optimal blob data 80 may indicate an optimal intensity characteristic of 90% of the full scale intensity value of the optical sensor 36 pixels for one tracker 38, an optimal intensity characteristic of 80% of the full scale intensity value of the optical sensor 36 pixels for another tracker 38, and so on.

Under this arrangement, responsive to receiving image data indicating blobs 124 corresponding to the active markers 54 of one or more trackers 38, the navigation controller 22 may be configured to assign the blobs 124 to the active markers 54 of each tracker 38 based on the one or more optimal characteristics specific to the tracker 38. More specifically, to determine whether a blob 124 corresponds to a given tracker 38, the navigation controller 22 may be configured to determine a difference between an acquired characteristic of the blob 124 and the corresponding optimal characteristic specific to the tracker 38, and to determine whether the difference is less than a threshold value (*e.g.,* 5% of the corresponding optimal characteristic). If so, then the navigation controller 22 may be configured to determine that the blob 124 corresponds to the tracker 38, and assign the blob 124 to the active marker 54 of the tracker 38 corresponding to the blob 124, such as based on the predefined geometry of markers 54 of the tracker 38 as described above.

In some examples, such as when characteristics of multiple types are acquired for each blob 124, the navigation controller 22 may be configured to determine whether a blob 124 corresponds to a given tracker 38 by determining whether each difference between an acquired characteristic of the blob 124 and the corresponding optimal characteristic specific to the tracker 38 is less than a threshold value determined based on the corresponding optimal characteristic (*e.g.,* 5% of the corresponding optimal characteristic). Alternatively, the navigation controller 22 may be configured to determine an average of the differences between or a sum of squared differences between the acquired characteristics of the blob 124 and the corresponding optimal characteristics specific to the tracker 38, and determine whether such value is less than a threshold value. If so, then the navigation controller 22 may be configured to determine that the blob 124 corresponds to the tracker 38, and to assign the blob 124 to the active marker 54 of the tracker 38 corresponding to the blob 124, as described above.

In some examples, the navigation controller 22 may be configured to determine one or more combined blob characteristics for a given set of blobs 124 identified as corresponding to a same active marker 54 as described above, and compare the combined blob characteristics to the corresponding optimal characteristics as described in the preceding paragraph to determine whether the set of blobs 124 corresponds to a given tracker 38. If so, then the navigation controller 22 may be configured to determine that the set of blobs 124 corresponds to the tracker 38, and assign the set of blobs 124 to the active marker 54 of the tracker 38 corresponding to the blobs 124, such as based on the predefined geometry of markers 54 of the tracker 38 as described above.

When the trackers 38 are optimized to different optimal characteristics, multiple trackers 38 may be present in the surgical workspace that have substantially equivalent predetermined geometries of markers 54. In other words, assuming a same pose in the surgical workspace and the same light emitting characteristics, the predetermined geometries of markers 54 of these trackers 38 may be indistinguishable by the navigation controller 22. Optimizing such trackers 38 to varying optimal characteristics may thus enable the navigation system 12 to distinguish between such trackers 38.

Responsive to determining the blobs 124 corresponding to the active markers 54 of a given tracker 38 based on the optimal characteristics specific to the tracker 38, the navigation controller 22 may be configured to track a pose of the tracker 38, and optimize the light signals emitted from the active markers 54 of the tracker 38 based on the optimal characteristics specific to the tracker 38, as described above.

In some examples, the navigation controller 22 may also or alternatively be configured to optimize the light signals emitted from the active markers 54 of the trackers 38 based on determined positions of the active markers 54 in the surgical workspace. More particularly, the navigation controller 22 may be configured to determine the position of each active marker 54 in the surgical workspace based on the image data as described above. Based on the determined positions of the active markers 54 in the surgical workspace and/or the optimal characteristics, the navigation controller 22 may be configured to communicate at least one control signal to the trackers 38 that cause the trackers 38 to adjust the light signal emitted from at least one of the active markers 54.

For instance, for each of the active markers 54 of a given tracker 38, the navigation controller 22 may be configured to compare one or more acquired characteristics of the blob 124 corresponding to the active marker 54 to the matching optimal characteristics to determine whether the blob 124 is suboptimal as described above. Responsive to determining that the blob 124 corresponding to the active marker 54 is suboptimal, the navigation controller 22 may be configured to communicate a control signal to the tracker 38 that causes the tracker 38 to adjust the light signal emitted from the active marker 54 based on the determined position of the active marker 54.

More particularly, the navigation controller 22 may be configured to compare a presently determined position of the given active marker 54 to a previously determined position of the active marker 54 in the surgical workspace to determine whether the distance between the active marker 54 and localizer camera 18 has changed, and if so, adjust the light signal emitted from the active marker 54. For instance, the navigation controller 22 may be configured to determine whether the change in distance indicates an increase or a decrease in the distance between the active marker 54 and the localizer camera 18. If the change in distance indicates an increase, then the navigation controller 22 may be configured to communicate a control signal to the tracker 38 that causes the tracker 38 to increase the intensity and/or duration of the light signal emitted from the active marker 54, and if the distance has decreased, then the navigation controller 22 may be configured to communicate a control signal to the tracker that causes tracker 38 to reduce the intensity and/or duration of the light signal emitted from the active marker 54. The extent to which the intensity and/or duration of the emitted light signal is increased or reduced may be proportional to the change in distance. In addition or alternatively, the navigation controller 22 may be configured to implement a PID loop and/or stored lookup tables to determine an extent by which to increase or reduce the intensity and/or duration of the emitted light signal based on the changed distance.

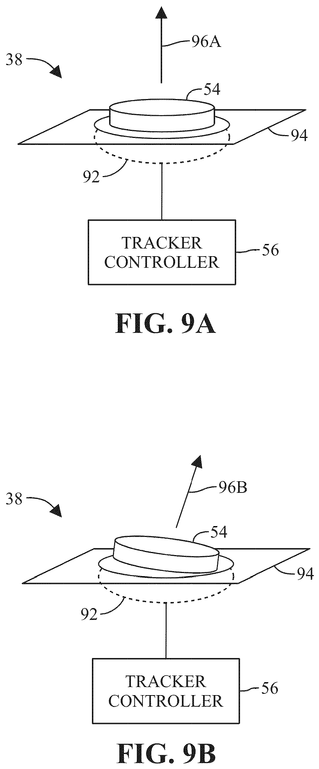

The navigation controller 22 may also or alternatively be configured to adjust the light signal emitted from at least one of the active markers 54 in the surgical workspace based on the comparison of the acquired characteristics of the blobs 124 corresponding to the active markers 54 to the optimal characteristics by being configured to reposition at least one of the active markers 54 based on the comparison. More particularly, referring to FIGS. 9A and 9B, each tracker 38 may include at least one actuator 92 for repositioning the active markers 54 of the tracker 38. For instance, as shown in the illustrated example, each marker 54 of a given tracker 38 may include a dedicated actuator 92 fixed to the marker 54 that is configured to rotate the marker 54 relative to a body 94 of the tracker 38 so as to aim the active marker 54. As the marker 54 is aimed further towards the localizer camera 18, more of the light signal emitted from the active marker 54 may be detected by the localizer camera 18, and as the marker 54 is aimed further away from the localizer camera 18, less of the light signal emitted from the active marker 54 may be detected by the localizer camera 18.

Each actuator 92 of a given tracker 38 may be communicatively coupled to and operated by the tracker controller 56 of the tracker 38. The navigation controller 22 may thus be configured to reposition an active marker 54 of a tracker 38 by communicating a control signal to the tracker controller 56 of the tracker 38, which in turn may vary the orientation of the marker 54 relative to the localizer camera 18 by operating the actuator 92 fixed to the active marker 54. For instance, FIGS. 9A and 9B illustrate an example in which the navigation controller 22 has caused the illustrated active marker 54 to change from facing in the direction represented by arrow 96A to facing in the direction represented by arrow 96B.

Thus, for each of the blobs 124 in the received image data corresponding to a given tracker 38, the navigation controller 22 may be configured to compare one or more acquired characteristics of the blob 124 to the corresponding optimal characteristics to determine whether the blob 124 is suboptimal as described above. Responsive to determining that the blob 124 is suboptimal based on the comparison, the navigation controller 22 may be configured to communicate a control signal to the tracker 38 that causes the tracker 38 to reposition the active marker 54 corresponding to the blob 124 for further iterations of tracking the active marker 54.

As an example, assuming the acquired characteristic of each blob 124 indicates an acquired value, and the corresponding optimal characteristic indicates at least one optimal value, for each blob 124, the navigation controller 22 may be configured to compare the acquired value indicated for the blob 124 to the at least one optimal value to determine whether the acquired value is greater than the at least one optimal value. Responsive to the comparison indicating that the acquired value for a blob 124 is greater than the at least one optimal value, the navigation controller 22 may be configured to communicate a control signal to the tracker 38 that causes the tracker 38 to reposition the active marker 54 corresponding to the blob 124 away from the localizer camera 18. Conversely, responsive to the comparison indicating that the acquired value for the blob 124 is less than the at least one optimal value, the navigation controller 22 may be configured to communicate a control signal to the tracker 38 that causes the tracker 38 to reposition the active marker 54 corresponding to the blob 124 towards the localizer camera 18.

The extent to which the active marker 54 is repositioned towards or away from the localizer camera 18 may be proportional to the difference between the acquired characteristic and optimal characteristic. In addition or alternatively, the navigation controller 22 may be configured to implement a PID loop and/or stored lookup tables to determine an extent by which to reposition the active marker 54 based on the difference between the acquired characteristic and optimal characteristic.

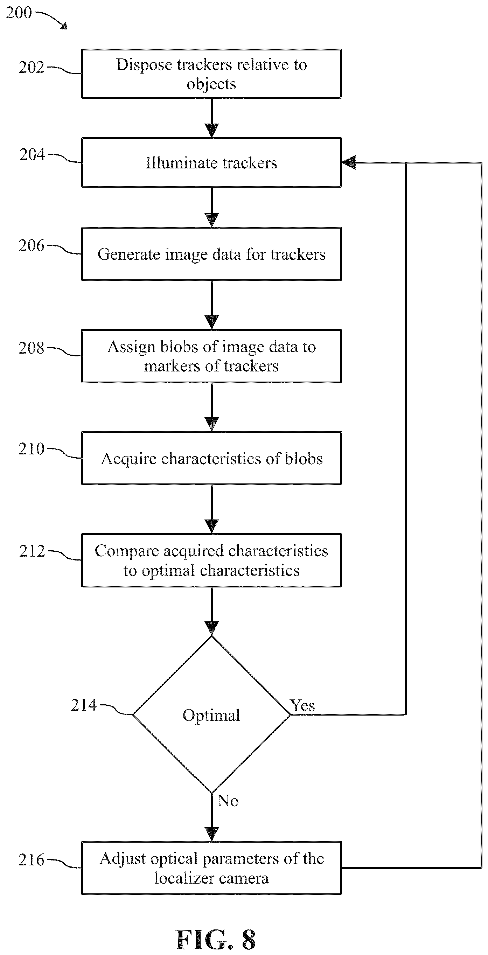

FIG. 8 illustrates another method 200 for optimizing tracking of an object in the surgical workspace by adjusting one or more optical parameters of the localizer camera 18. The method 200 may be utilized when passive trackers 38 including passive markers 54 are present in the surgical workspace. The method 200 may be facilitated by the surgical navigation system 12, or more particularly by the navigation controller 22, such as upon execution of the software 64. For the sake of efficiency, certain details of the blocks of method 200 that may correspond to the blocks of method 100 already described above are not repeated in the forthcoming paragraphs.

In block 202, trackers 38 may be disposed relative to objects to be tracked. Each tracker 38 may include a predetermined geometry of passive markers 54. In block 204, the trackers 38 may be illuminated. More particularly, the navigation controller 22 may be configured to communicate a control signal to the localizer controller 52 that causes the localizer controller 52 to emit a light signal into the surgical workspace from the light source 58. In block 206, image data may be generated based on the reflections of the emitted light signal by the passive markers 54. Specifically, the localizer controller 52 may generate image data for each optical sensor 36 representative of an image indicating a blob 124 corresponding to each of the passive markers 54 generated from a reflection by the passive marker 54 of the emitted light signal. The pixel coordinates of each blob 124 within the image data for each optical sensor 36 may correspond to the position on the image plane of the optical sensor 36 in which a reflection was detected. In block 208, each blob 124 indicated in the image data may be assigned to the passive marker 54 of the trackers 38 corresponding to the blob 124, such as using the triangulation and matching method described above.

In block 210, one or more characteristics of each blob 124 may be acquired. For instance, the navigation controller 22 may be configured to acquire an intensity characteristic, and/or a size characteristic, and/or a shape characteristic for each blob 124. Thereafter, in block 212, the acquired blob characteristics may be compared to one or more optimal blob characteristics, such as those indicated in the optimal blob data 80 stored in the non-volatile storage 62 of the navigation controller 22. In block 214, a determination may be made of whether the blobs 124 are optimal based on the comparison.

The navigation controller 22 may be configured to compare the acquired blob characteristics to the optimal blob characteristics by combining the acquired blob characteristics of a same type (e.g., intensity, size, shape) to form a combined blob characteristic for the characteristic type. For instance, relative to the blob intensity type characteristic, the navigation controller 22 may be configured to calculate an average of the intensity values indicated by the acquired intensity characteristics of the blobs 124 as the combined blob characteristic for the intensity type characteristic. Relative to the blob size type characteristic, the navigation controller 22 may be configured to calculate an average of the areas indicated by the acquired size characteristics of the blobs 124 as the combined blob characteristic for the blob size type characteristic. Relative to the blob shape type characteristic, the navigation controller 22 may be configured to calculate an average of the ratios indicated by the acquired shape characteristics of the blobs 124 as the combined blob characteristic for the blob shape type characteristic. Thereafter, the navigation controller 22 may be configured to compare the combined blob characteristics to their corresponding optimal blob characteristics to determine whether the combined blob characteristics are optimal as described above.

Responsive to determining that a combined blob characteristic of a given type is suboptimal ("No" branch of block 214), in block 216, at least one optical parameter of the localizer camera 18 may be adjusted. In one example, the light signal emitted from the light source 58 may be adjusted so as to cause the passive markers 54 to convey light signals in future tracking iterations that result in generation of a combined blob characteristic of the type that is optimal or closer to optimal. More specifically, the navigation controller 22 may be configured to adjust an intensity and/or duration of the light signal emitted from the light source 58, such as by communicating a control signal to the localizer controller 52 that causes the localizer controller 52 to adjust the current applied to the light source 58 as described above.

As an example, if a combined blob characteristic indicates a value greater than the one or more optimal values defined by the corresponding optimal blob characteristic, then the navigation controller 22 may be configured to communicate a control signal to the localizer controller 52 that causes the localizer controller 52 to decrease the intensity and/or duration of the light signal emitted from the light source 58. Conversely, if the combined blob characteristic indicates a value that is less than the one or more optimal values defined by the corresponding optimal blob characteristic, then the navigation controller 22 may be configured to communicate a control signal to the localizer controller 52 that causes the localizer controller 52 to increase the intensity and/or duration of the light signal emitted from the light source 58.

The extent to which the intensity and/or duration of the emitted light signal is increased or reduced may be proportional to the difference between the acquired characteristic and the optimal characteristic. In addition or alternatively, the navigation controller 22 may be configured to implement a PID loop and/or stored lookup tables to determine an extent by which to increase or reduce the intensity and/or duration of the emitted light signal so as to make the acquired blob characteristic optimal.

Similar to that described above in connection with the active markers 54, the navigation controller 22 may be configured to prioritize the optimization of certain types of combined blob characteristics over others. For instance, the navigation controller 22 may be configured to first optimize the combined intensity characteristic. Responsive to the combined intensity characteristic becoming optimized, the navigation controller 22 may be configured to optimize the combined size characteristic. Responsive to the combined size characteristic becoming optimized, the navigation controller 22 may be configured to optimize the combined shape characteristic. During each optimization iteration, the navigation controller 22 may be configured to acquire and check whether a type of combined blob characteristic of highest priority is optimal. If not, then the navigation controller 22 may be configured to adjust at least one optical parameter of the localizer camera 18 to optimize the type of combined blob characteristic as described above. If the type of combined blob characteristic of the highest priority is determined optimal, then the navigation controller 22 may be configured to determine and check whether the type of combined blob characteristic of the next highest priority is optimal, and so on.

In some instances, the navigation controller 22 may be configured to track and optimize the passive trackers 38 independently by emitting varying light signals from the light source 58, with each emitted light signal having at least one characteristic corresponding to a different tracker 38 in the surgical workspace. In other words, each emitted light signal corresponding to a different tracker 38 may have at least one characteristic, such as a light intensity characteristic and/or a light duration characteristic, that differs from that of the emitted light signals corresponding to the other trackers 38 in the surgical workspace.

Based on the varying poses of the trackers 38 in the surgical workspace, the characteristics of the blobs 124 generated by one of the trackers 38 to an emitted light signal may vary from the characteristics of the blobs 124 generated by the other trackers 38 to the same light signal. Correspondingly, different trackers 38 may generate optimal blobs responsive to emitted light signals of different characteristics. For instance, one tracker 38 may generate optimal blobs 124 when the light signal emitted from the light source 58 is at 90% of the full intensity level of the light source 58, another tracker 38 may generate optimal blobs 124 when the light signal emitted from the light source 58 is at 80% of the full intensity level of the light source 58, and so on.

The navigation controller 22 may thus be configured to track and optimize tracking of the trackers 38 by alternating between emitting light signals from the light source 58 having varying characteristics, such as having varying intensity levels ranging from 60% to 95%, and receiving image data from the localizer camera 18 corresponding to each emitted light signal that indicates a blob 124 for each of the passive markers 54 generated from a reflection by the passive marker 54 of the emitted light signal. Although each instance of received image data may include a blob 124 generated by each passive marker 54 of each tracker 38, the blobs 124 corresponding to the passive markers 54 of one tracker 38 may be closer to optimal than the blobs 124 corresponding to the passive markers 54 of the other trackers 38 based on the poses of the trackers 38 in the surgical workspace and the characteristics of the emitted light signal.

Accordingly, for each tracker 38, the navigation controller 22 may be configured to acquire a characteristic of each blob 124 in each received instance of image data that corresponds to a marker 54 of the tracker 38, and to compare the acquired characteristics to the optimal characteristics to determine which of the instances of received image data is closest to optimal. Responsive to determining the instance of received image data closest to optimal, the navigation controller 22 may be configured to assign the characteristics of the light signal corresponding to the instance of received image data to the tracker 38, and to perform future iterations of tracking a pose of the tracker 38 in the surgical workspace based on the light signal characteristics assigned to the tracker 38.

Thus, each tracker 38 may be assigned specific light characteristics, and to track a pose of a given tracker 38, the navigation controller 22 may be configured to emit a light signal from the light source 58 specific to the tracker 38, such as by emitting a light signal having the light characteristics assigned to the tracker 38. The navigation controller 22 may then be configured to track a pose the tracker 38 based on the blobs 124 indicated in the image data received for the emitted light signal specific to the tracker 38, as described above.

The navigation controller 22 may also be configured to differentiate the blobs 124 corresponding to the passive markers 54 of one tracker 38 from those corresponding to the passive markers 54 of the other trackers 38 based on the lighting characteristics assigned to the one tracker 38 and the one or more stored optimal characteristics. More specifically, responsive to receiving image data corresponding to a light signal emitted from the light source 58 with at least one characteristic corresponding to a given tracker 38, the navigation controller 22 may be configured to differentiate the blobs 124 corresponding to the given tracker 38 from the other trackers 38 in the surgical workspace by acquiring at least one characteristic of each blob 124 indicated by the image data, comparing the acquired characteristics of the blobs 124 to the one or more optimal characteristics, and differentiating the blobs 124 based on the comparison.

For example, for each of the blobs 124 indicated by the image data, the navigation controller 22 may be configured to determine a difference between the one or more acquired characteristics of the blob 124 and the corresponding one or more optimal characteristics, such as by calculating an average of the differences or a sum of squared differences. Thereafter, the navigation controller 22 may be configured to determine whether the determined difference is less than a threshold value, and if so, to determine that the blob 124 corresponds to the given tracker 38. In alternative examples, the navigation controller 22 may be configured to determine that the blob 124 corresponds to the given tracker 38 responsive to determining that each difference between an acquired characteristic of the blob 124 and the corresponding optimal characteristic is less than a threshold value.

Responsive to differentiating the blobs 124 corresponding to the given tracker 38, the navigation controller 22 may be configured to adjust the characteristics of the emitted light signal assigned to the given tracker 38 so as to optimize tracking of the given tracker 38 as described above. In a next iteration of tracking and/or optimizing tracking of the given tracker 38, the navigation controller 22 may be configured to utilize the adjusted characteristics. Similar to that described above, when the trackers 38 are tracked and optimized using light signals emitted from the light source 58 having varying characteristics, multiple trackers 38 may be present in the surgical workspace that have substantially equivalent predetermined geometries of passive markers 54.

In some examples, the navigation controller 22 may also be configured to adjust the at least one optical parameter of the localizer camera 18 based on the tracked poses of the trackers 38 in the surgical workspace. More particularly, the navigation controller 22 may be configured to determine the position of each passive marker 54 in the surgical workspace based on received image data as described above, which in turn may indicate the poses of the trackers 38 in the surgical workspace. Based on the determined poses, the navigation controller 22 may be configured to adjust the at least one optical parameter of the localizer camera 18. For instance, responsive to comparing the acquired characteristics of the blobs 124 to the optimal characteristics and determining that the blobs 124 are suboptimal, the navigation controller 22 may be configured to adjust the at least one optical parameter of the localizer camera 18 based on the determined positions of the passive markers 54.

In one example, the navigation controller 22 may be configured to adjust the at least one optical parameter of the localizer camera 18 based on the determined positions of the passive markers 54 by being configured to determine an average distance between the passive markers 54 of the one or more trackers 38 and the localizer camera 18, and to compare this average difference to a previously calculated average distance for the passive markers 54 to determine a change in the average distance between the passive markers 54 and the localizer camera 18. The navigation controller 22 may then be configured to adjust the at least one optical parameter of the localizer camera 18 based on the change in average distance.

For instance, the navigation controller 22 may be configured to determine whether the change in average distance indicates an increase or a decrease in the average distance between the passive markers 54 and the localizer camera 18. Responsive to the change in distance indicating an increase in the average distance between the passive markers 54 and the localizer camera 18, the navigation controller 22 may be configured to increase an intensity and/or duration of the light signal emitted from the light source 58 to illuminate the passive markers 54. Conversely, responsive to the change in distance indicating a decrease in the average distance between the passive markers 54 and the localizer camera 18, the navigation controller 22 may be configured to reduce an intensity and/or duration of the light signal emitted from the light source 58 to illuminate the passive markers 54. The extent to which the intensity and/or duration of the emitted light signal is increased or reduced may be proportional to the change in average distance. In addition or alternatively, the navigation controller 22 may be configured to implement a PID loop and/or stored lookup tables to determine an extent by which to increase or reduce the intensity and/or duration of the emitted light signal so as to make the acquired blob characteristic optimal based on the change in average distance.

In some examples, in addition or alternatively to adjusting the light signal emitted from the light source 58, the navigation controller 22 may be configured to adjust other optical parameters of the localizer camera 18 to optimize the blobs 124 generated from the markers 54. For instance, the navigation controller 22 may be configured to, based on the comparison of the one or more acquired characteristics of the blobs 124 to the one or more optimal characteristics, adjust an electronic aperture time of each optical sensor 36 of the localizer camera 18. More particularly, the navigation controller 22 may be configured to form one or more combined blob characteristics for each optical sensor 36 from the image data generated for the optical sensor 36 as described above, and for each combined blob characteristic, compare the value indicated by the combined blob characteristic to the optimal value indicated by the corresponding optimal blob characteristic. Responsive to the comparison indicating that the value of the combined blob characteristic is greater than the optimal value, the navigation controller 22 may be configured to reduce the electronic aperture time of the corresponding optical sensor 36, and responsive to the comparison indicating that the value of the combined blob characteristic is less than the optimal value, the navigation controller 22 may be configured to increase the electronic aperture time of the corresponding optical sensor 36.

As further examples, the localizer camera 18 may also include a mechanical shutter and/or mechanical aperture for each optical sensor 36, and the navigation controller 22 may be configured to, based on the comparison of the one or more acquired characteristics of the blobs 124 to the one or more optimal characteristics, adjust a shutter time of the mechanical shutter and/or adjust a capture size of the mechanical aperture for each optical sensor 36. More particularly, the navigation controller 22 may be configured to form one or more combined blob characteristics for each optical sensor 36 from the image data generated for the optical sensor 36 as described above, and for each combined blob characteristic, compare the value indicated by the combined blob characteristic to the optimal value indicated by the corresponding optimal blob characteristic. Responsive to the comparison indicating that the value of the combined blob characteristic is greater than the optimal value, the navigation controller 22 may be configured to reduce the shutter time of the mechanical shutter and/or the capture size of the mechanical aperture for the optical sensor 36, and responsive to the comparison indicating that the value of the combined blob characteristic is less than the optimal value, the navigation controller 22 may be configured to increase the shutter time of the mechanical shutter and/or the capture size of the mechanical aperture for the optical sensor 36.

Referring again to FIG. 8, responsive to determining that each of the combined blob characteristics is optimal ("Yes" branch of block 214), or to adjusting at least one optical parameter of the localizer camera 18 in block 216, the method 200 may return to block 204 to again illuminate the trackers 38 via the light source 58 of the localizer camera 18.

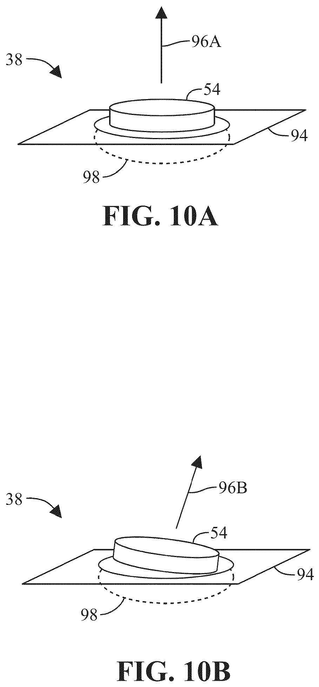

In some examples, the passive markers 54 of each tracker 38 may be manually repositionable, and the navigation controller 22 may also be configured to, based on the comparison of the acquired characteristics of the blobs 124 to the optimal characteristics, determine and display guidance for repositioning at least one passive marker 54 of the trackers 38, such as on the displays 28, 30. For instance, referring to FIGS. 10A and 10B, each passive marker 54 of a given tracker 38 may be seated in a rotatable socket 98 that allows a user to manually rotate the passive marker 54 relative to the body 94 of the tracker 38 so as to aim the passive marker 54 towards and away from the localizer camera 18.

Thus, for each of the blobs 124 indicated by received image data, the navigation controller 22 may be configured to assign the blob 124 to the passive marker 54 corresponding to the blob 124, compare the one or more acquired characteristics of the blob 124 to the one or more optimal corresponding optimal characteristics to determine whether the blob 124 is suboptimal, and responsive to determining that the blob 124 is suboptimal based on the comparison, determine and display guidance for repositioning the passive marker 54 corresponding to the blob 124.

For instance, assuming an acquired characteristic of each blob 124 indicates an acquired value, and a corresponding optimal characteristic indicates an optimal value, for each blob the navigation controller 22 may be configured to assign the blob 124 to the passive marker 54 corresponding to the blob 124, and compare the acquired value indicated for the blob 124 to the optimal value. Responsive to the comparison indicating that the acquired value for the blob 124 is greater than the optimal value, the navigation controller 22 may be configured to determine and display guidance to reposition the passive marker 54 corresponding to the blob 124 away from the localizer camera 18. Conversely, responsive to the comparison indicating that the acquired value for the blob 124 is less than the optimal value, the navigation controller 22 may be configured to determine and display guidance to reposition the passive marker 54 corresponding to the blob 124 towards the localizer camera 18.

Some surgical environments may incorporate both passive and active trackers 38. In this case, the navigation controller 22 may be configured to implement both the above-described processes for optimizing the active trackers 38 and the above-described processes for optimizing the passive trackers 38. In one example, the navigation controller 22 may be configured to alternate between optimizing and tracking the active and passive trackers 38 using the above described processes. Alternatively, the navigation controller 22 may be configured to implement both tracking and optimizing processes simultaneously, such as by causing the markers 54 of the active trackers 38 to emit light signals at a different frequency as the light signals emitted from the light source 58 to reduce interference and improve differentiation between the tracker 38 types, and/or by utilizing varying sets of one or more optimal blob characteristics for the different tracker types to further facilitate such differentiation.

In general, the routines executed to implement aspects of foregoing description, whether implemented as part of an operating system or a specific application, component, program, object, module or sequence of instructions, or even a subset thereof, may be referred to herein as "computer program code," or simply "program code." Program code may comprise computer readable instructions that are resident at various times in various memory and storage devices in a computer and that, when read and executed by one or more processors in a computer, cause that computer to perform the operations necessary to execute operations and/or elements embodying the various aspects of the description. Computer readable program instructions for carrying out operations of the various aspects of the description may be, for example, assembly language or either source code or object code written in any combination of one or more programming languages.

The program code embodied in any of the applications/modules described herein may be capable of being individually or collectively distributed as a program product in a variety of different forms. In particular, the program code may be distributed using a computer readable storage medium having computer readable program instructions thereon for causing a processor to carry out aspects of the description.

Computer readable storage media, which is inherently non-transitory, may include volatile and non-volatile, and removable and non-removable tangible media implemented in any method or technology for storage of information, such as computer-readable instructions, data structures, program modules, or other data. Computer readable storage media may further include random access memory (RAM), read-only memory (ROM), erasable programmable read-only memory (EPROM), electrically erasable programmable read-only memory (EEPROM), flash memory or other solid state memory technology, portable compact disc read-only memory (CD-ROM), or other optical storage, magnetic cassettes, magnetic tape, magnetic disk storage or other magnetic storage devices, or any other medium that can be used to store the desired information and which can be read by a computer. A computer readable storage medium should not be construed as transitory signals per se (e.g., radio waves or other propagating electromagnetic waves, electromagnetic waves propagating through a transmission media such as a waveguide, or electrical signals transmitted through a wire). Computer readable program instructions may be downloaded to a computer, another type of programmable data processing apparatus, or another device from a computer readable storage medium or to an external computer or external storage device via a network.

Computer readable program instructions stored in a computer readable medium may be used to direct a computer, other types of programmable data processing apparatus, or other devices to function in a particular manner, such that the instructions stored in the computer readable medium produce an article of manufacture including instructions that implement the functions/acts specified in the flowcharts, sequence diagrams, and/or block diagrams. The computer program instructions may be provided to one or more processors such that the instructions, which execute via the one or more processors, cause a series of computations to be performed to implement the functions and/or acts specified in the flowcharts, sequence diagrams, and/or block diagrams described herein.

In certain alternatives, the functions and/or acts specified in the flowcharts, sequence diagrams, and/or block diagrams may be re-ordered, processed serially, and/or processed concurrently without departing from the scope of the invention. Moreover, any of the flowcharts, sequence diagrams, and/or block diagrams may include more or fewer blocks than those illustrated herein.

The terminology used herein is for the purpose of describing particular examples only and is not intended to be limiting. As used herein, the singular forms "a," "an," and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. Furthermore, to the extent that the terms "includes," "having," "has," "with," "comprised of," or variants thereof are used in either the detailed description or the claims, such terms are intended to be inclusive in a manner similar to the term "comprising."

While a description of various examples has been provided and while these examples have been described in considerable detail, it is not the intention of the Applicant to restrict or in any way limit the scope of the appended claims to such detail. Additional advantages and modifications will readily appear to those skilled in the art. The invention in its broader aspects is therefore not limited to the specific details, representative apparatus and method, and illustrative examples shown and described. Accordingly, departures may be made from such details without departing from the spirit or scope of the Applicant's general inventive concept.
The present disclosure also provides for the following examples:
1. A navigation system for optimizing tracking of an object in a surgical workspace, the navigation system comprising:
   a tracker disposed relative to the object and including a predefined geometry of active markers for tracking a pose of the tracker in the surgical workspace;
   a localizer camera configured to cooperate with the tracker to generate image data indicating a blob for each of the active markers generated from a light signal emitted from the active marker; and
   a controller communicatively coupled to the tracker and the localizer camera, the controller being configured to:
      assign each of the blobs to the active marker corresponding to the blob;
      acquire a characteristic of each blob;
      compare the acquired characteristics to an optimal characteristic; and
      based on the comparison, communicate at least one control signal to the tracker that causes the tracker to adjust the light signal emitted from at least one of the active markers.
2. The navigation system of example 1, wherein the at least one control signal communicated to the tracker causes the tracker to adjust an intensity and/or duration of the light signal emitted from the at least one of the active markers.
3. The navigation system of example 1 or 2, wherein for each of the blobs the controller is configured to:
   compare the acquired characteristic of the blob to the optimal characteristic to determine whether the blob is suboptimal; and
   responsive to determining that the blob is suboptimal based on the comparison,
   communicate a control signal to the tracker that causes the tracker to adjust the light signal emitted from the active marker corresponding to the blob.
4. The navigation system of any one of the preceding examples, wherein the acquired characteristic of each blob indicates a first value, the optimal characteristic indicates a second value, and for each blob the controller is configured to:
   compare the first value indicated for the blob to the second value;
   responsive to the comparison indicating that the first value for the blob is greater than the second value, communicate a control signal to the tracker that causes the tracker to reduce an intensity and/or duration of the light signal emitted from the active marker corresponding to the blob; and
   responsive to the comparison indicating that the first value for the blob is less than the second value, communicate a control signal to the tracker that causes the tracker to increase the intensity and/or duration of the light signal emitted from the active marker corresponding to the blob.
5. The navigation system of any one of the preceding examples, wherein the acquired characteristics are blob intensity characteristics, and the optimal characteristic is an optimal blob intensity characteristic.
6. The navigation system of any one of examples 1-4, wherein the acquired characteristics are blob size characteristics, and the optimal characteristic is an optimal blob size characteristic.
7. The navigation system of any one of examples 1-4, wherein the acquired characteristics are blob shape characteristics, and the optimal characteristic is an optimal blob shape characteristic.
8. The navigation system of any one of examples 1-4, wherein the acquired characteristics are defined as acquired first characteristics, the optimal characteristic is defined as a first optimal characteristic, and the controller is configured to:
   acquire one or more second characteristics of one or more of the blobs;
   compare the one or more acquired second characteristics to a second optimal characteristic; and
   based on the comparison of the one or more acquired second characteristics to the second optimal characteristic, communicate at least one control signal to the tracker that causes the tracker to adjust the light signal emitted from at least one of the one or more active markers corresponding to the one or more blobs.
9. The navigation system of any one of examples 1-4 and 8, wherein the acquired characteristics are defined as acquired first characteristics, the optimal characteristic is defined as a first optimal characteristic, and for each blob the controller is configured to:
   compare the acquired first characteristic of the blob to the first optimal characteristic to determine whether the acquired first characteristic of the blob is suboptimal;
   responsive to determining that the acquired first characteristic of the blob is suboptimal based on the comparison, communicate a control signal to the tracker that causes the tracker to adjust the light signal emitted from the active marker corresponding to the blob; and
   responsive to determining that the acquired first characteristic of the blob is not suboptimal based on the comparison:
      acquire a second characteristic of the blob;
      compare the acquired second characteristic of the blob to a second optimal characteristic to determine whether the acquired second characteristic of the blob is suboptimal; and
      responsive to determining that the acquired second characteristic of the blob is suboptimal based on the comparison, communicate a control signal to the tracker that causes the tracker to adjust the light signal emitted from the active marker corresponding to the blob.
10. The navigation system of example 8 or 9, wherein the acquired first characteristics are blob intensity characteristics, and the acquired second characteristics are blob size characteristics or blob shape characteristics.
11. The navigation system of example 1 or 2, wherein the image data comprises first image data corresponding to a first optical sensor of the localizer camera and second image data corresponding to a second optical sensor of the localizer camera, each of the first and second image data indicating a blob for each active marker generated from a light signal emitted from the active marker, and the controller is configured to:
   identify a first blob from the first image data and a second blob from the second image data that correspond to a same active marker;
   acquire a first characteristic of the first blob and a second characteristic of the second blob;
   combine the acquired first characteristic and the acquired second characteristic to form a combined blob characteristic;
   compare the combined blob characteristic to the optimal characteristic to determine if the combined blob characteristic is suboptimal; and
   responsive to determining that the combined blob characteristic is suboptimal based on the comparison, communicate a control signal to the tracker that causes the tracker to adjust the light signal emitted from the active marker corresponding to the first and second blobs.
12. The navigation system of example 11, wherein the acquired first and second characteristics are acquired intensity characteristics, and the optimal characteristic is an optimal blob intensity characteristic.
13. The navigation system of example 5 or 12, wherein the optimal blob intensity characteristic indicates an intensity value greater than or equal to 75% and less than or equal to 95% of a full scale intensity value of the localizer camera.
14. The navigation system of example 11, wherein the combined blob characteristic is defined as a first combined blob characteristic, the optimal characteristic is defined as a first optimal characteristic, and the controller is configured to:
   acquire a third characteristic of the first blob and a fourth characteristic of the second blob;
   combine the acquired third characteristic and the acquired fourth characteristic to form a second combined blob characteristic;
   compare the second combined blob characteristic to a second optimal characteristic; and
   based on the comparison of the second combined blob characteristic to the second optimal characteristic, communicate a control signal to the tracker that causes the tracker to adjust the light signal emitted from the active marker corresponding to the first and second blobs.
15. The navigation system of example 11 or 14, wherein the combined blob characteristic is defined as a first combined blob characteristic, the optimal characteristic is defined as a first optimal characteristic, and the controller is configured to:
   compare the first combined blob characteristic to the first optimal characteristic to determine whether the first combined blob characteristic is suboptimal;
   responsive to determining that the first combined blob characteristic is suboptimal, communicate the control signal to the tracker that causes the tracker to adjust the light signal emitted from the active marker corresponding to the first and second blobs; and
   responsive to determining that the first combined blob characteristic is not suboptimal based on the comparison:
      acquire a third characteristic of the first blob and a fourth characteristic of the second blob;
      combine the acquired third characteristic and the acquired fourth characteristic to form a second combined blob characteristic;
      compare the second combined blob characteristic to a second optimal characteristic to determine whether the second combined blob characteristic is suboptimal; and
      responsive to determining that the second combined blob characteristic is suboptimal based on the comparison, communicate a control signal to the tracker that causes the tracker to adjust the light signal emitted from the active marker corresponding to the first and second blobs.
16. The navigation system of example 14 or 15, wherein the acquired first and second characteristics are blob intensity characteristics, and the acquired third and fourth characteristics are blob size characteristics or blob shape characteristics.
17. The navigation system of any one of the preceding examples, wherein the object is defined as a first object, the blobs are defined as first blobs, the tracker is defined as a first tracker, the acquired characteristics are defined as acquired first characteristics, the optimal characteristic is defined as a first optimal characteristic specific to the first tracker, and further comprising a second tracker disposed relative to a second object in the surgical workspace and including a predefined geometry of active markers for tracking a pose of the second tracker in the surgical workspace, wherein the image data generated by the localizer camera includes a second blob for each of the active markers of the second tracker generated from a light signal emitted from the active marker of the second tracker, and the controller is configured to:
   assign each of the second blobs to the active marker of the second tracker corresponding to the second blob;
   acquire a second characteristic of each second blob;
   compare the acquired second characteristics to a second optimal characteristic that is specific to the second tracker and differs from the first optimal characteristic; and
   based on the comparison, communicate at least one control signal to the second tracker that causes the second tracker to adjust the light signal emitted from at least one of the active markers of the second tracker.
18. The navigation system of example 17, wherein the controller is configured to assign the first blobs to the active markers of the first tracker based on the first optimal characteristic, and/or to assign the second blobs to the active markers of the second tracker based on the second optimal characteristic.
19. The navigation system of example 17 or 18, wherein for each of the first blobs the controller is configured to:
   determine a difference between the acquired first characteristic of the first blob and the first optimal characteristic;
   determine whether the difference between the acquired first characteristic of the first blob and the first optimal characteristic is less than a threshold value; and
   responsive to determining that the difference between the acquired first characteristic of the first blob and the first optimal characteristic is less than the threshold value, determine that the first blob corresponds to the first tracker and assign the first blob to the active marker of the first tracker corresponding to the first blob.
20. The navigation system of any one of examples 17-19, wherein the predefined geometry of active markers of the first tracker and the predefined geometry of active markers of the second tracker are substantially equivalent.
21. The navigation system of any one of the preceding examples, wherein the controller is configured to:
   determine positions of the active markers of the tracker in the surgical workspace based on the image data; and
   based on the determined positions of the active markers, communicate the at least one control signal to the tracker that causes the tracker to adjust the light signal emitted from at least one of the active markers.
22. The navigation system of example 21, wherein for each of the active markers the controller is configured to:
   compare the acquired characteristic of the blob corresponding to the active marker to the optimal characteristic to determine whether the blob corresponding to the active marker is suboptimal; and
   responsive to determining that the blob corresponding to the active marker is suboptimal, communicate a control signal to the tracker that causes the tracker to adjust the light signal emitted from the active marker based on the determined position of the active marker.
23. The navigation system of example 22, wherein the controller is configured to communicate a control signal to the tracker that causes the tracker to adjust the light signal emitted from the active marker based on the determined position of the active marker by being configured to:
   compare the determined position of the active marker to a previously determined position of the active marker to determine a change in distance between the active marker and the localizer camera; and
   based on the change in distance, communicate a control signal to the tracker that causes the tracker to adjust the light signal emitted from the active marker.
24. The navigation system of example 23, wherein the controller is configured to communicate a control signal to the tracker that causes the tracker to adjust the light signal emitted from the active marker based on the determined change in distance by being configured to:
   determine whether the change in distance indicates an increase or a decrease in the distance between the active marker and the localizer camera;
   responsive to the change in distance indicating an increase in the distance between the active marker and the localizer camera, communicate a control signal to the tracker that causes the tracker to increase an intensity and/or duration of the light signal emitted from the active marker; and
   responsive to the change in distance indicating a decrease in the distance between the active marker and the localizer camera, communicate a control signal to the tracker that causes the tracker to reduce an intensity and/or duration of the light signal emitted from the active marker.
25. A navigation system for optimizing tracking of objects in a surgical workspace, the navigation system comprising:
   a first tracker disposed relative to a first object in the surgical workspace and including a predefined geometry of active markers for tracking a pose of the first tracker in the surgical workspace;
   a second tracker disposed relative to a second object in the surgical workspace and including a predefined geometry of active markers for tracking a pose of the second tracker in the surgical workspace;
   a localizer camera configured to cooperate with the first and second trackers to generate image data indicating a first blob for each of the active markers of the first tracker generated from a light signal emitted from the active marker and a second blob for each of the active markers of the second tracker generated from a light signal emitted from the active marker; and
   a controller communicatively coupled to the first and second trackers and the localizer camera, the controller being configured to:
      acquire a characteristic of each of the first and second blobs;
      compare the acquired characteristics to a first optimal characteristic specific to the first tracker and a second optimal characteristic specific to the second tracker that differs from the first optimal characteristic; and
      based on the comparison, assign the first blobs to the first tracker and the second blobs to the second tracker.
26. A navigation system for optimizing tracking of an object in a surgical workspace, the navigation system comprising:
   a tracker disposed relative to the object and including a predefined geometry of active markers for tracking a pose of the tracker in the surgical workspace;
   a localizer camera configured to cooperate with the tracker to generate image data indicating a blob for each of the active markers generated from a light signal emitted from the active marker; and
   a controller communicatively coupled to the tracker and the localizer camera, the controller being configured to:
      determine positions of the active markers of the tracker in the surgical workspace based on the image data; and
      based on the determined positions of the active markers, communicate at least one control signal to the tracker that causes the tracker to adjust the light signal emitted from at least one of the active markers based on the determined positions.
27. A navigation system for optimizing tracking of an object in a surgical workspace, the navigation system comprising:
   a tracker disposed relative to the object and including a predefined geometry of passive markers for tracking a pose of the tracker in the surgical workspace;
   a localizer camera including a light source configured to emit a light signal for illuminating the passive markers, the localizer camera being configured to generate image data indicating a blob for each of the passive markers generated from a reflection by the passive marker of the light signal emitted from the light source; and
   a controller communicatively coupled to the localizer camera, the controller being configured to:
      acquire a characteristic of each blob;
      compare the acquired characteristics to an optimal characteristic; and
      based on the comparison, adjust at least one optical parameter of the localizer camera.
28. The navigation system of example 27, wherein the controller is configured to adjust at least one optical parameter of the localizer camera based on the comparison by being configured to adjust an intensity and/or duration of the light signal emitted from the light source to illuminate the passive markers based on the comparison.
29. The navigation system of example 27 or 28, wherein the controller is configured to:
   combine the acquired characteristics to form a combined blob characteristic;
   compare the combined blob characteristic to the optimal characteristic to determine whether the combined blob characteristic is suboptimal; and
   responsive to determining that the combined blob characteristic is suboptimal based on the comparison, adjust the at least one optical parameter of the localizer camera.
30. The navigation system of any one of examples 27-29, wherein the acquired characteristics are defined as acquired first characteristics, the combined blob characteristic is defined as a first combined blob characteristic, the optimal characteristic is defined as a first optimal characteristic, and the controller is configured to:
   compare the first combined blob characteristic to the first optimal characteristic to determine whether the first combined blob characteristic is suboptimal;
   responsive to determining that the first combined blob characteristic is suboptimal based on the comparison, adjust the at least one optical parameter of the localizer camera; and
   responsive to determining that the first combined blob characteristic is not suboptimal based on the comparison:
      acquire a second characteristic of each blob;
      combine the acquired second characteristics to form a second combined blob characteristic;
      compare the second combined blob characteristic to a second optimal characteristic to determine whether the second combined blob characteristic is suboptimal; and
      responsive to determining that the second combined blob characteristic is suboptimal based on the comparison, adjust the at least one optical parameter of the localizer camera.
31. The navigation system of any one of examples 27-30, wherein the object is defined as a first object, the blobs are defined as first blobs, the tracker is defined as a first tracker, the light signal is defined as a first light signal specific to the first tracker, and further comprising a second tracker disposed relative to a second object in the surgical workspace and including a predefined geometry of passive markers for tracking a pose of the second tracker in the surgical workspace, wherein the controller is configured to:
   emit a second light signal specific to the second tracker from the light source, the second light signal having at least one characteristic that differs from at least one corresponding characteristic of the first light signal;
   receive image data corresponding to the second light signal generated by the localizer camera, the received image data indicating a second blob for each of the passive markers of the second tracker generated from a reflection by the passive marker of the second light signal emitted from the light source;
   acquire a characteristic of each second blob;
   compare the acquired characteristics of the second blobs to the optimal characteristic to determine whether the acquired characteristics of the second blobs are suboptimal; and
   responsive to determining that the acquired characteristics of the second blobs are suboptimal based on the comparison, adjust the at least one characteristic of the second light signal.
32. The navigation system of example 31, wherein the at least one characteristic of the second light signal that differs from the at least one corresponding characteristic of the first light signal comprises a light intensity characteristic and/or light duration characteristic.
33. The navigation system of example 31 or 32, wherein the image data corresponding to the second light signal indicates a third blob for each of the passive markers of the first tracker generated from a reflection by the passive marker of the second light signal emitted from the light source, and the controller is configured to, responsive to receiving the image data corresponding to the second light signal, differentiate the second blobs from the third blobs based on the optimal characteristic.
34. The navigation system of any one of examples 31-33, wherein the predefined geometry of passive markers of the first tracker and the predefined geometry of passive markers of the second tracker are substantially equivalent.
35. The navigation system of any one of examples 27-34, whereon the controller is configured to:
   emit light signals from the light source having varying characteristics;
   receive image data generated by the localizer camera for each of the emitted light signals that indicates a blob for each of the passive markers generated from a reflection by the passive marker of the emitted light signal;
   for each instance of received image data, acquire a characteristic of each blob indicated by the image data and compare the acquired characteristics to the optimal characteristic to determine which of the instances of received image data is closest to optimal;
   responsive to determining the instance of received image data closest to optimal, assign the characteristics of the light signal corresponding to the instance of received image data to the tracker; and
   track a pose of the tracker in the surgical workspace based on the light signal characteristics assigned to the tracker.
36. The navigation system of example 35, wherein the controller is configured to:
   emit a light signal from the light source having the light signal characteristics assigned to the tracker to illuminate the passive markers of the tracker;
   receive image data corresponding to the emitted light signal having the light signal characteristics assigned to the tracker, the received image data indicating a blob for each passive marker of the tracker generated from a reflection of the emitted light signal having the light signal characteristics assigned to the tracker by the passive marker;
   acquire a characteristic of each of the blobs in the received image data;
   compare the acquired characteristics of the blobs in the received image data to the optimal characteristic to determine whether the acquired characteristics of the blobs are suboptimal; and
   responsive to determining that the acquired characteristics of the blobs are suboptimal based on the comparison, adjust the light signal characteristics assigned to the tracker.
37. The navigation system of any one of examples 27-36, wherein the controller is configured to:
   determine positions of the passive markers of the tracker in the surgical workspace based on the image data; and
   based on the determined positions of the passive markers, adjust the at least one optical parameter of the localizer camera.
38. The navigation system of any one of examples 27-37, wherein the controller is configured to adjust the at least one optical parameter of the localizer camera based on the comparison by being configured to adjust an electronic aperture time of the localizer camera based on the comparison.
39. The navigation system of any one of examples 27-38, wherein the localizer camera includes a mechanical shutter, and the controller is configured to adjust the at least one optical parameter of the localizer camera based on the comparison by being configured to adjust a shutter time of the mechanical shutter based on the comparison.
40. The navigation system of any one of examples 27-39, wherein the localizer camera includes a mechanical aperture, and the controller is configured to adjust the at least one optical parameter of the localizer camera based on the comparison by being configured to adjust a capture size of the mechanical aperture based on the comparison.
41. A navigation system for tracking objects in a surgical workspace, the navigation system comprising:
   a first tracker disposed relative to a first object in the surgical workspace and including a predefined geometry of passive markers for tracking a pose of the first tracker in the surgical workspace;
   a second tracker disposed relative to a second object in the surgical workspace and including a predefined geometry of passive markers for tracking a pose of the second tracker in the surgical workspace;
   a localizer camera including a light source configured to emit a light signal for illuminating the passive markers of the first and second trackers, the localizer camera being configured to generate image data indicating a blob for each of the passive markers of the first and second trackers generated from a reflection by the passive marker of the light signal emitted from the light source; and
   a controller communicatively coupled to the localizer camera, the controller being configured to:
      emit a first light signal from the light source that is specific to the first tracker;
      receive image data generated by the localizer camera corresponding to the emitted first light signal;
      track a pose of the first tracker in the surgical workspace based on the received image data corresponding to the first light signal;
      emit a second light signal from the light source specific to the second tracker and having at least one characteristic that differs from at least one corresponding characteristic of the first light signal;
      receive image data generated by the localizer camera corresponding to the emitted second light signal; and
      track a pose of the second tracker in the surgical workspace based on the received image data corresponding to the second light signal.
42. A navigation system for optimizing tracking of an object in a surgical workspace, the navigation system comprising:
   a tracker disposed relative to the object and including a predefined geometry of passive markers for tracking a pose of the tracker in the surgical workspace;
   a localizer camera including a light source configured to emit a light signal for illuminating the passive markers, the localizer camera being configured to generate image data indicating a blob for each of the passive markers generated from a reflection by the passive marker of the light signal emitted from the light source; and
   a controller communicatively coupled to the localizer camera, the controller being configured to:
      emit light signals from the light source having varying characteristics;
      receive image data generated by the localizer camera for each of the emitted light signals that indicates a blob for each of the passive markers generated from a reflection by the passive marker of the emitted light signal;
      for each instance of received image data, acquire a characteristic of each blob indicated by the image data and compare the acquired characteristics to an optimal characteristic to determine which of the instances of received image data is closest to optimal;
      responsive to determining the instance of received image data closest to optimal, assign the characteristics of the light signal corresponding to the instance of received image data to the tracker; and
      track a pose of the tracker in the surgical workspace based on the light signal characteristics assigned to the tracker.
43. A navigation system for optimizing tracking of an object in a surgical workspace, the navigation system comprising:
   a tracker disposed relative to the object and including a predefined geometry of passive markers for tracking a pose of the tracker in the surgical workspace;
   a localizer camera including a light source configured to emit a light signal for illuminating the passive markers, the localizer camera being configured to generate image data indicating a blob for each of the passive markers generated from a reflection by the passive marker of the light signal emitted from the light source; and
   a controller communicatively coupled to the localizer camera, the controller being configured to:
      determine positions of the passive markers of the tracker in the surgical workspace based on the image data; and
      based on the determined positions of the passive markers, adjust at least one optical parameter of the localizer camera.
44. A navigation system for optimizing tracking of an object in a surgical workspace, the navigation system comprising:
   a tracker disposed relative to the object and including a predefined geometry of manually repositionable passive markers for tracking a pose of the tracker in the surgical workspace;
   a localizer camera including a light source configured to emit a light signal for illuminating the passive markers, the localizer camera being configured to generate image data indicating a blob for each of the passive markers generated from a reflection by the passive marker of the light signal emitted from the light source; and
   a controller communicatively coupled to the localizer camera, the controller being configured to:
      acquire a characteristic of each blob;
      compare the acquired characteristics to an optimal characteristic; and
      determine and display guidance for repositioning the passive markers of the tracker based on the comparison.
45. A method for optimizing tracking of an object in a surgical workspace by a navigation system including a tracker disposed relative to the object and including a predefined geometry of active markers for tracking a pose of the tracker in the surgical workspace, a localizer camera configured to cooperate with the tracker to generate image data indicating a blob for each of the active markers generated from a light signal emitted from the active marker, and a controller communicatively coupled to the tracker and the localizer camera, the controller being configured to:
   disposing the tracker relative to the object in the surgical workspace;
   generating, by the localizer camera, the image data indicating a blob for each of the active markers generated from a light signal emitted from the active marker assigning, by the controller, each of the blobs to the active marker corresponding to the blob;
   acquiring, by the controller, a characteristic of each blob;
   comparing, by the controller, the acquired characteristics to an optimal characteristic; and
   based on the comparison, communicating, by the controller, at least one control signal to the tracker that causes the tracker to adjust the light signal emitted from at least one of the active markers.
46. The method of example 45, wherein the at least one control signal communicated to the tracker causes the tracker to adjust an intensity and/or duration of the light signal emitted from the at least one of the active markers.
47. The method of example 45 or 46, further comprising:
   comparing the acquired characteristic of each of the blobs to the optimal characteristic to determine whether the blob is suboptimal;
   identifying one or more of the blobs as suboptimal based on the comparison; and
   for each of the identified one or more blobs, communicating a control signal to the tracker that causes the tracker to adjust the light signal emitted from the active marker corresponding to the blob.
48. The method of any one of examples 45-47, wherein the acquired characteristic of each blob indicates a first value, the optimal characteristic indicates a second value, and further comprising:
   comparing the first value indicated for each of the blobs to the second value to determine whether the first value is greater than the second value;
   identifying one or more of the blobs each for which the first value is greater than the second value based on the comparison; and
   for each of the identified one or more blobs for which the first value is greater than the second value, communicating a control signal to the tracker that causes the tracker to reduce an intensity and/or duration of the light signal emitted from the active marker corresponding to the blob.
49. The method of any one of examples 45-48, wherein the acquired characteristic of each blob indicates a first value, the optimal characteristic indicates a second value, and further comprising:
   comparing the first value indicated for each of the blobs to the second value to determine whether the first value is less than the second value;
   identifying one or more of the blobs each for which the first value is less than the second value based on the comparison; and
   for each of the identified one or more blobs for which the first value is less than the second value, communicating a control signal to the tracker that causes the tracker to increase an intensity and/or duration of the light signal emitted from the active marker corresponding to the blob.
50. The method of any one of examples 45-49, wherein the acquired characteristics are blob intensity characteristics, and the optimal characteristic is an optimal blob intensity characteristic.
51. The method of any one of examples 45-49, wherein the acquired characteristics are blob size characteristics, and the optimal characteristic is an optimal blob size characteristic.
52. The method of any one of examples 45-49, wherein the acquired characteristics are blob shape characteristics, and the optimal characteristic is an optimal blob shape characteristic.
53. The method of any one of examples 45-49, wherein the acquired characteristics are defined as acquired first characteristics, the optimal characteristic is defined as a first optimal characteristic, and curter comprising:
   acquiring one or more second characteristics of one or more of the blobs;
   comparing the one or more acquired second characteristics to a second optimal characteristic; and
   based on the comparison of the one or more acquired second characteristics to the second optimal characteristic, communicating at least one control signal to the tracker that causes the tracker to adjust the light signal emitted from at least one of the one or more active markers corresponding to the one or more blobs.
54. The method of any one of examples 45-49 and 53, wherein the acquired characteristics are defined as acquired first characteristics, the optimal characteristic is defined as a first optimal characteristic, and further comprising:
   comparing the acquired first characteristic of each of the blobs to the first optimal characteristic to determine whether the acquired first characteristic of the blob is suboptimal;
   identifying, from the blobs, one or more first blobs each for which the first optimal characteristic is not suboptimal based on the comparison of the acquired first characteristic to the first optimal characteristic;
   acquiring a second characteristic of each of the one or more first blobs;
   comparing the acquired second characteristic of each of the one or more first blobs to a second optimal characteristic to determine whether the acquired second characteristic of the blob is suboptimal;
   identifying, from the one or more first blobs, one or more second blobs each for which the second optimal characteristic is suboptimal based on the comparison of the acquired second characteristic to the second optimal characteristic; and
   for each of the identified one or more second blobs, communicating a control signal to the tracker that causes the tracker to adjust the light signal emitted from the active marker corresponding to the blob.
55. The method of example 53 or 54, wherein the acquired first characteristics are blob intensity characteristics, and the acquired second characteristics are blob size characteristics or blob shape characteristics.
56. The method of example 45 or 46, wherein the image data comprises first image data corresponding to a first optical sensor of the localizer camera and second image data corresponding to a second optical sensor of the localizer camera, each of the first and second image data indicating a blob for each active marker generated from a light signal emitted from the active marker, and further comprising:
   for each of the active markers:
   identifying a first blob from the first image data and a second blob from the second image data that correspond to the active marker;
   acquiring a first characteristic of the first blob and a second characteristic of the second blob;
   combining the acquired first characteristic and the acquired second characteristic to form a combined blob characteristic; and
   comparing the combined blob characteristic to the optimal characteristic to determine if the combined blob characteristic is suboptimal;
   identifying one or more of the combined blob characteristics as suboptimal based on the comparison of each of the combined blob characteristics to the optimal characteristic; and
   for each of the identified one or more combined blob characteristics, communicating a control signal to the tracker that causes the tracker to adjust the light signal emitted from the active marker corresponding to the combined blob characteristic.
57. The method of example 56, wherein the acquired first and second characteristics are acquired intensity characteristics, and the optimal characteristic is an optimal blob intensity characteristic.
58. The method of example 50 or 57, wherein the optimal blob intensity characteristic indicates an intensity value greater than or equal to 75% and less than or equal to 95% of a full scale intensity value of the localizer camera.
59. The method of example 56, wherein the combined blob characteristics are defined as first combined blob characteristics, the optimal characteristic is defined as a first optimal characteristic, and the controller is configured to:
   for each of one or more of the active markers:
   acquiring a third characteristic of the first blob and a fourth characteristic of the second blob corresponding to the active marker;
   combining the acquired third characteristic and the acquired fourth characteristic to form a second combined blob characteristic; and
   comparing the second combined blob characteristic to a second optimal characteristic; and
   based on the comparison of the second combined blob characteristic for each of the one or more active markers to the second optimal characteristic, communicating a control signal to the tracker that causes the tracker to adjust the light signal emitted from at least one of the one or more active markers.
60. The method of example 56 or 59, wherein the combined blob characteristics are defined as a first combined blob characteristics, the optimal characteristic is defined as a first optimal characteristic, and further comprising:
   identifying one or more of the first combined blob characteristics as not suboptimal based on the comparison of each of the one or more first combined blob characteristics to the optimal characteristic; and
   for each of the one or more first combined blob characteristics identified as not suboptimal:
      acquiring a third characteristic of the first blob and a fourth characteristic of the second blob corresponding to the first combined blob characteristic;
      combining the acquired third characteristic and the acquired fourth characteristic to form a second combined blob characteristic;
      comparing the second combined blob characteristic to a second optimal characteristic to determine whether the second combined blob characteristic is suboptimal; and
      responsive to determining that the second combined blob characteristic is suboptimal based on the comparison, communicating a control signal to the tracker that causes the tracker to adjust the light signal emitted from the active marker corresponding to the first combined blob characteristic.
61. The method of example 59 or 60, wherein the acquired first and second characteristics are blob intensity characteristics, and the acquired third and fourth characteristics are blob size characteristics or blob shape characteristics.
62. The method of any one of examples 45-61, wherein the object is defined as a first object, the blobs are defined as first blobs, the tracker is defined as a first tracker, the acquired characteristics are defined as acquired first characteristics, the optimal characteristic is defined as a first optimal characteristic specific to the first tracker, and further comprising:
   disposing a second tracker relative to a second object in the surgical workspace, the second tracker including a predefined geometry of active markers for tracking a pose of the second tracker in the surgical workspace, wherein the image data generated by the localizer camera includes a second blob for each of the active markers of the second tracker generated from a light signal emitted from the active marker of the second tracker;
   assigning each of the second blobs to the active marker of the second tracker corresponding to the second blob;
   acquiring a second characteristic of each second blob;
   comparing the acquired second characteristics to a second optimal characteristic that is specific to the second tracker and differs from the first optimal characteristic; and
   based on the comparison of the acquired second characteristics to a second optimal characteristic, communicating at least one control signal to the second tracker that causes the second tracker to adjust the light signal emitted from at least one of the active markers of the second tracker.
63. The method of example 62, further comprising assigning the first blobs to the active markers of the first tracker based on the first optimal characteristic and/or assigning the second blobs to the active markers of the second tracker based on the second optimal characteristic.
64. The method of example 62 or 63, further comprising:
   for each of the first blobs:
   determining a difference between the acquired first characteristic of the first blob and the first optimal characteristic;
   determining that the difference between the acquired first characteristic of the first blob and the first optimal characteristic is less than a threshold value; and
   responsive to determining that the difference between the acquired first characteristic of the first blob and the first optimal characteristic is less than the threshold value, determining that the first blob corresponds to the first tracker and
   assigning the first blob to the active marker of the first tracker corresponding to the first blob.
65. The method of any one of examples 61-64, wherein the predefined geometry of active markers of the first tracker and the predefined geometry of active markers of the second tracker are substantially equivalent.
66. The method of any one of examples 45-65, further comprising:
   determining positions of the active markers of the tracker in the surgical workspace based on the image data; and
   based on the determined positions of the active markers, communicating the at least one control signal to the tracker that causes the tracker to adjust the light signal emitted from at least one of the active markers.
67. The method of example 66, further comprising:
   comparing the acquired characteristic of each of the blobs to the optimal characteristic to determine whether the blob is suboptimal;
   identifying one or more of the blobs as suboptimal based on the comparison; and
   for each of the one or more blobs identified as suboptimal, communicating a control signal to the tracker that causes the tracker to adjust the light signal emitted from the active marker corresponding to the blob based on the determined position of the active marker.
68. The method of example 67, wherein communicating a control signal to the tracker that causes the tracker to adjust the light signal emitted from the active marker corresponding to the blob based on the determined position of the active marker comprises:
   comparing the determined position of the active marker to a previously determined position of the active marker to determine a change in distance between the active marker and the localizer camera; and
   based on the change in distance, communicating a control signal to the tracker that causes the tracker to adjust the light signal emitted from the active marker.
69. The method of example 68, wherein communicating a control signal to the tracker that causes the tracker to adjust the light signal emitted from the active marker based on the change in distance comprises:
   determining that the change in distance indicates an increase in the distance between the active marker and the localizer camera; and
   responsive to determining that the change in distance indicates an increase in the distance between the active marker and the localizer camera, communicating a control signal to the tracker that causes the tracker to increase an intensity and/or duration of the light signal emitted from the active marker.
70. A method for optimizing tracking of objects in a surgical workspace by a navigation system including a first tracker disposed relative to a first object in the surgical workspace and including a predefined geometry of active markers for tracking a pose of the first tracker in the surgical workspace, a second tracker disposed relative to a second object in the surgical workspace and including a predefined geometry of active markers for tracking a pose of the second tracker in the surgical workspace, a localizer camera configured to cooperate with the first and second trackers to generate image data indicating a first blob for each of the active markers of the first tracker generated from a light signal emitted from the active marker and a second blob for each of the active markers of the second tracker generated from a light signal emitted from the active marker; and a controller communicatively coupled to the first and second trackers and the localizer camera, the method comprising:
   deposing the first and second trackers relative to the first and second objects in the surgical workspace;
   generating, by the localizer camera, the image data indicating a first blob for each of the active markers of the first tracker generated from a light signal emitted from the active marker and a second blob for each of the active markers of the second tracker generated from a light signal emitted from the active marker;
   acquiring, by the controller, a characteristic of each of the first and second blobs;
   comparing, by the controller, the acquired characteristics to a first optimal characteristic specific to the first tracker and a second optimal characteristic specific to the second tracker that differs from the first optimal characteristic; and
   based on the comparison, assigning, by the controller, the first blobs to the first tracker and the second blobs to the second tracker.
71. A method for optimizing tracking of an object in a surgical workspace by a navigation system including a tracker disposed relative to the object and including a predefined geometry of active markers for tracking a pose of the tracker in the surgical workspace, a localizer camera configured to cooperate with the tracker to generate image data indicating a blob for each of the active markers generated from a light signal emitted from the active marker, and a controller communicatively coupled to the tracker and the localizer camera, the method comprising:
   disposing the tracker relative to the object in the surgical workspace;
   generating, by the localizer camera, the image data indicating a blob for each of the active markers generated from a light signal emitted from the active marker;
   determining, by the controller, positions of the active markers of the tracker in the surgical workspace based on the image data; and
   based on the determined positions of the active markers, communicating at least one control signal to the tracker that causes the tracker to adjust the light signal emitted from at least one of the active markers based on the determined positions.
72. A method for optimizing tracking of an object in a surgical workspace by a navigation system including a tracker disposed relative to the object and including a predefined geometry of passive markers for tracking a pose of the tracker in the surgical workspace, a localizer camera including a light source configured to emit a light signal for illuminating the passive markers, the localizer camera being configured to generate image data indicating a blob for each of the passive markers generated from a reflection by the passive marker of the light signal emitted from the light source, and a controller communicatively coupled to the localizer camera, the method comprising:
   disposing a tracker relative to the object in the surgical workspace;
   generating, by the localizer camera, the image data indicating a blob for each of the passive markers generated from a reflection by the passive marker of the light signal emitted from the light source;
   acquiring, by the controller, a characteristic of each blob;
   comparing, by the controller, the acquired characteristics to an optimal characteristic; and
   based on the comparison, adjusting, by the controller, at least one optical parameter of the localizer camera.
73. A method for tracking objects in a surgical workspace by a navigation system including a first tracker disposed relative to a first object in the surgical workspace and including a predefined geometry of passive markers for tracking a pose of the first tracker in the surgical workspace, a second tracker disposed relative to a second object in the surgical workspace and including a predefined geometry of passive markers for tracking a pose of the second tracker in the surgical workspace, a localizer camera including a light source configured to emit a light signal for illuminating the passive markers of the first and second trackers, the localizer camera being configured to generate image data indicating a blob for each of the passive markers of the first and second trackers generated from a reflection by the passive marker of the light signal emitted from the light source, and a controller communicatively coupled to the localizer camera, the method comprising:
   disposing the first and second trackers relative to the first and second objects respectively in the surgical workspace;
   emitting, from the light source, a first light signal that is specific to the first tracker; receiving, by the controller, image data generated by the localizer camera corresponding to the emitted first light signal;
   tracking, by the controller, a pose of the first tracker in the surgical workspace based on the received image data corresponding to the first light signal;
   emitting, from the light source, a second light signal that is specific to the second tracker and has at least one characteristic that differs from at least one corresponding characteristic of the first light signal;
   receiving, by the controller, image data generated by the localizer camera corresponding to the emitted second light signal; and
   tracking, by the controller, a pose of the second tracker in the surgical workspace based on the received image data corresponding to the second light signal.
74. A method for optimizing tracking of an object in a surgical workspace by a navigation system including a tracker disposed relative to the object and including a predefined geometry of passive markers for tracking a pose of the tracker in the surgical workspace, a localizer camera including a light source configured to emit a light signal for illuminating the passive markers, the localizer camera being configured to generate image data indicating a blob for each of the passive markers generated from a reflection by the passive marker of the light signal emitted from the light source, and a controller communicatively coupled to the localizer camera, the method comprising:
   disposing the tracker relative to the object in the surgical workspace;
   emitting, from the light source, light signals having varying characteristics;
   receiving, by the controller, image data generated by the localizer camera for each of the emitted light signals that indicates a blob for each of the passive markers generated from a reflection by the passive marker of the emitted light signal;
   for each instance of received image data, acquiring, by the controller, a characteristic of each blob indicated by the image data and comparing, by the controller, the acquired characteristics to an optimal characteristic to determine which of the instances of received image data is closest to optimal;
   responsive to determining the instance of received image data closest to optimal, assigning, by the controller, the characteristics of the light signal corresponding to the instance of received image data to the tracker; and
   tracking, by the controller, a pose of the tracker in the surgical workspace based on the light signal characteristics assigned to the tracker.
75. A method for optimizing tracking of an object in a surgical workspace by a navigation system including a tracker disposed relative to the object and including a predefined geometry of passive markers for tracking a pose of the tracker in the surgical workspace, a localizer camera including a light source configured to emit a light signal for illuminating the passive markers, the localizer camera being configured to generate image data indicating a blob for each of the passive markers generated from a reflection by the passive marker of the light signal emitted from the light source, and a controller communicatively coupled to the localizer camera, the method comprising:
   disposing the tracker relative to the object in the surgical workspace;
   generating, by the localizer camera, image data indicating a blob for each of the passive markers generated from a reflection by the passive marker of the light signal emitted from the light source;
   determining, by the controller, the positions of the passive markers of the tracker in the surgical workspace based on the image data; and
   based on the determined positions of the passive markers, adjusting, by the controller, at least one optical parameter of the localizer camera.
76. A method for optimizing tracking of an object in a surgical workspace by a navigation system, the navigation system including a tracker disposed relative to the object and including a predefined geometry of manually repositionable passive markers for tracking a pose of the tracker in the surgical workspace, a localizer camera including a light source configured to emit a light signal for illuminating the passive markers, the localizer camera being configured to generate image data indicating a blob for each of the passive markers generated from a reflection by the passive marker of the light signal emitted from the light source, and a controller communicatively coupled to the localizer camera, the method comprising:
   disposing the tracker relative to the object in the surgical workspace;
   generating, by the localizer camera, the image data indicating a blob for each of the passive markers generated from a reflection by the passive marker of the light signal emitted from the light source;
   acquiring, by the controller, a characteristic of each blob;
   comparing, by the controller, the acquired characteristics to an optimal characteristic; and
   determining and displaying, by the controller, guidance for repositioning the passive markers of the tracker based on the comparison.

## Claims

1. A navigation system (12) for optimizing tracking of an object in a surgical workspace, the navigation system (12) comprising:
a tracker (38) disposed relative to the object and including a predefined geometry of active markers (54) for tracking a pose of the tracker (38) in the surgical workspace;
a localizer camera (18) configured to cooperate with the tracker (38) to generate image data indicating a blob for each of the active markers (54) generated from a light signal emitted from the active marker (54); and
a controller (22) communicatively coupled to the tracker (38) and the localizer camera (18), the controller (22) being configured to:
assign each of the blobs to the active marker (54) corresponding to the blob;
acquire a characteristic of each blob;
compare the acquired characteristics to an optimal characteristic; and
based on the comparison, communicate at least one control signal to the tracker (38) that causes the tracker to adjust the light signal emitted from at least one of the active markers (54).

2. The navigation system (12) of claim 1, wherein the at least one control signal communicated to the tracker (38) causes the tracker (38) to adjust an intensity and/or duration of the light signal emitted from the at least one of the active markers (54).

3. The navigation system of claim 1 or 2, wherein
{i} for each of the blobs the controller (22) is configured to:
compare the acquired characteristic of the blob to the optimal characteristic to determine whether the blob is suboptimal; and
responsive to determining that the blob is suboptimal based on the comparison, communicate a control signal to the tracker (38) that causes the tracker (38) to adjust the light signal emitted from the active marker (54) corresponding to the blob; and/or
{ii} the acquired characteristic of each blob indicates a first value, the optimal characteristic indicates a second value, and for each blob the controller (22) is configured to:
compare the first value indicated for the blob to the second value;
responsive to the comparison indicating that the first value for the blob is greater than the second value, communicate a control signal to the tracker (38) that causes the tracker (38) to reduce an intensity and/or duration of the light signal emitted from the active marker corresponding to the blob; and
responsive to the comparison indicating that the first value for the blob is less than the second value, communicate a control signal to the tracker (38) that causes the tracker (38) to increase the intensity and/or duration of the light signal emitted from the active marker (38) corresponding to the blob.

4. The navigation system (12) of any one of the preceding claims, wherein the acquired characteristics are blob intensity characteristics, and the optimal characteristic is an optimal blob intensity characteristic.

5. The navigation system (12) of any one of claims 1-3, wherein
{iii} the acquired characteristics are blob size characteristics, and the optimal characteristic is an optimal blob size characteristic; or
{iv} the acquired characteristics are blob shape characteristics, and the optimal characteristic is an optimal blob shape characteristic.

6. The navigation system (12) of any one of claims 1-3, wherein the acquired characteristics are defined as acquired first characteristics, the optimal characteristic is defined as a first optimal characteristic, and the controller (22) is configured to:
acquire one or more second characteristics of one or more of the blobs;
compare the one or more acquired second characteristics to a second optimal characteristic; and
based on the comparison of the one or more acquired second characteristics to the second optimal characteristic, communicate at least one control signal to the tracker that causes the tracker (38) to adjust the light signal emitted from at least one of the one or more active markers (54) corresponding to the one or more blobs; wherein, optionally, the acquired first characteristics are blob intensity characteristics, and the acquired second characteristics are blob size characteristics or blob shape characteristics.

7. The navigation system (12) of any one of claims 1-3 and 6, wherein the acquired characteristics are defined as acquired first characteristics, the optimal characteristic is defined as a first optimal characteristic, and for each blob the controller (22) is configured to:
compare the acquired first characteristic of the blob to the first optimal characteristic to determine whether the acquired first characteristic of the blob is suboptimal;
responsive to determining that the acquired first characteristic of the blob is suboptimal based on the comparison, communicate a control signal to the tracker (38) that causes the tracker (38) to adjust the light signal emitted from the active marker (54) corresponding to the blob; and
responsive to determining that the acquired first characteristic of the blob is not suboptimal based on the comparison:
acquire a second characteristic of the blob;
compare the acquired second characteristic of the blob to a second optimal characteristic to determine whether the acquired second characteristic of the blob is suboptimal; and
responsive to determining that the acquired second characteristic of the blob is suboptimal based on the comparison, communicate a control signal to the tracker (38) that causes the tracker (38) to adjust the light signal emitted from the active marker (54) corresponding to the blob; wherein, optionally, the acquired first characteristics are blob intensity characteristics, and the acquired second characteristics are blob size characteristics or blob shape characteristics.

8. The navigation system (12) of claim 1 or 2, wherein the image data comprises first image data corresponding to a first optical sensor (36) of the localizer camera (18) and second image data corresponding to a second optical sensor (36) of the localizer camera (18), each of the first and second image data indicating a blob for each active marker (54) generated from a light signal emitted from the active marker (54), and the controller (22) is configured to:
identify a first blob from the first image data and a second blob from the second image data that correspond to a same active marker (54);
acquire a first characteristic of the first blob and a second characteristic of the second blob;
combine the acquired first characteristic and the acquired second characteristic to form a combined blob characteristic;
compare the combined blob characteristic to the optimal characteristic to determine if the combined blob characteristic is suboptimal; and
responsive to determining that the combined blob characteristic is suboptimal based on the comparison, communicate a control signal to the tracker (38) that causes the tracker (38) to adjust the light signal emitted from the active marker (54) corresponding to the first and second blobs.

9. The navigation system (12) of claim 8, wherein the acquired first and second characteristics are acquired intensity characteristics, and the optimal characteristic is an optimal blob intensity characteristic.

10. The navigation system (12) of claim 4 or 9, wherein the optimal blob intensity characteristic indicates an intensity value greater than or equal to 75% and less than or equal to 95% of a full scale intensity value of the localizer camera (18).

11. The navigation system (12) of claim 8, wherein the combined blob characteristic is defined as a first combined blob characteristic, the optimal characteristic is defined as a first optimal characteristic, and the controller (22) is configured to:
acquire a third characteristic of the first blob and a fourth characteristic of the second blob;
combine the acquired third characteristic and the acquired fourth characteristic to form a second combined blob characteristic;
compare the second combined blob characteristic to a second optimal characteristic; and
based on the comparison of the second combined blob characteristic to the second optimal characteristic, communicate a control signal to the tracker that causes the tracker to adjust the light signal emitted from the active marker (54) corresponding to the first and second blobs; wherein, optionally, the acquired first and second characteristics are blob intensity characteristics, and the acquired third and fourth characteristics are blob size characteristics or blob shape characteristics.

12. The navigation system (12) of claim 8 or 11, wherein the combined blob characteristic is defined as a first combined blob characteristic, the optimal characteristic is defined as a first optimal characteristic, and the controller (22) is configured to:
compare the first combined blob characteristic to the first optimal characteristic to determine whether the first combined blob characteristic is suboptimal;
responsive to determining that the first combined blob characteristic is suboptimal, communicate the control signal to the tracker (38) that causes the tracker (38) to adjust the light signal emitted from the active marker (54) corresponding to the first and second blobs; and
responsive to determining that the first combined blob characteristic is not suboptimal based on the comparison:
acquire a third characteristic of the first blob and a fourth characteristic of the second blob;
combine the acquired third characteristic and the acquired fourth characteristic to form a second combined blob characteristic;
compare the second combined blob characteristic to a second optimal characteristic to determine whether the second combined blob characteristic is suboptimal; and
responsive to determining that the second combined blob characteristic is suboptimal based on the comparison, communicate a control signal to the tracker (38) that causes the tracker (38) to adjust the light signal emitted from the active marker (54) corresponding to the first and second blobs; wherein, optionally, the acquired first and second characteristics are blob intensity characteristics, and the acquired third and fourth characteristics are blob size characteristics or blob shape characteristics.

13. The navigation system (12) of any one of the preceding claims, wherein the object is defined as a first object, the blobs are defined as first blobs, the tracker (38) is defined as a first tracker, the acquired characteristics are defined as acquired first characteristics, the optimal characteristic is defined as a first optimal characteristic specific to the first tracker, and further comprising a second tracker disposed relative to a second object in the surgical workspace and including a predefined geometry of active markers (54) for tracking a pose of the second tracker in the surgical workspace, wherein the image data generated by the localizer camera (18) includes a second blob for each of the active markers (54) of the second tracker generated from a light signal emitted from the active marker (54) of the second tracker, and the controller (22) is configured to:
assign each of the second blobs to the active marker (54) of the second tracker corresponding to the second blob;
acquire a second characteristic of each second blob;
compare the acquired second characteristics to a second optimal characteristic that is specific to the second tracker and differs from the first optimal characteristic; and
based on the comparison, communicate at least one control signal to the second tracker that causes the second tracker to adjust the light signal emitted from at least one of the active markers (54) of the second tracker; wherein, optionally, the controller (22) is configured to assign the first blobs to the active markers of the first tracker based on the first optimal characteristic, and/or to assign the second blobs to the active markers of the second tracker based on the second optimal characteristic.

14. The navigation system of claim 13, wherein
{v} for each of the first blobs the controller (22) is configured to:
determine a difference between the acquired first characteristic of the first blob and the first optimal characteristic;
determine whether the difference between the acquired first characteristic of the first blob and the first optimal characteristic is less than a threshold value; and
responsive to determining that the difference between the acquired first characteristic of the first blob and the first optimal characteristic is less than the threshold value, determine that the first blob corresponds to the first tracker and assign the first blob to the active marker of the first tracker corresponding to the first blob; and/or
{vi} the predefined geometry of active markers (54) of the first tracker and the predefined geometry of active markers (54) of the second tracker are substantially equivalent.

15. The navigation system of any one of the preceding claims, wherein the controller (22) is configured to:
determine positions of the active markers (54) of the tracker (38) in the surgical workspace based on the image data; and
based on the determined positions of the active markers, communicate the at least one control signal to the tracker (38) that causes the tracker (38) to adjust the light signal emitted from at least one of the active markers (54); wherein, optionally, for each of the active markers (54) the controller (22) is configured to:
compare the acquired characteristic of the blob corresponding to the active marker (54) to the optimal characteristic to determine whether the blob corresponding to the active marker (54) is suboptimal; and
responsive to determining that the blob corresponding to the active marker is suboptimal, communicate a control signal to the tracker (38) that causes the tracker (38) to adjust the light signal emitted from the active marker (54) based on the determined position of the active marker (54); wherein, further optionally, the controller (22) is configured to communicate a control signal to the tracker (38) that causes the tracker (38) to adjust the light signal emitted from the active marker (54) based on the determined position of the active marker (54) by being configured to:
compare the determined position of the active marker (54) to a previously determined position of the active marker (54) to determine a change in distance between the active marker (54) and the localizer camera (18); and
based on the change in distance, communicate a control signal to the tracker (38) that causes the tracker (38) to adjust the light signal emitted from the active marker (54); wherein, still further optionally, the controller (22) is configured to communicate a control signal to the tracker (38) that causes the tracker (38) to adjust the light signal emitted from the active marker (54) based on the determined change in distance by being configured to:
determine whether the change in distance indicates an increase or a decrease in the distance between the active marker (54) and the localizer camera (18);
responsive to the change in distance indicating an increase in the distance between the active marker (54) and the localizer camera (18), communicate a control signal to the tracker (38) that causes the tracker (38) to increase an intensity and/or duration of the light signal emitted from the active marker (54); and
responsive to the change in distance indicating a decrease in the distance between the active marker (54) and the localizer camera (18), communicate a control signal to the tracker (38) that causes the tracker (38) to reduce an intensity and/or duration of the light signal emitted from the active marker (54).
